(19) Europäisches Patentamt / European Patent Office / Office européen des brevets

(11) **EP 3 915 468 A1**

(12) **EUROPEAN PATENT APPLICATION**
published in accordance with Art. 153(4) EPC

(43) Date of publication:
**01.12.2021 Bulletin 2021/48**

(51) Int Cl.:
*A61B 5/02* (2006.01)       *A61B 5/022* (2006.01)
*A61B 5/145* (2006.01)

(21) Application number: **20744802.8**

(22) Date of filing: **06.01.2020**

(86) International application number:
**PCT/JP2020/000059**

(87) International publication number:
**WO 2020/153108 (30.07.2020 Gazette 2020/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.01.2019   JP 2019008542**
**14.05.2019   JP 2019091652**

(71) Applicant: **Kyocera Corporation**
**Kyoto-shi, Kyoto 612-8501 (JP)**

(72) Inventor: **AJIMA Hiromi**
**Kyoto-shi, Kyoto 612-8501 (JP)**

(74) Representative: **Viering, Jentschura & Partner mbB**
**Patent- und Rechtsanwälte**
**Am Brauhaus 8**
**01099 Dresden (DE)**

(54) **ELECTRONIC DEVICE, ELECTRONIC DEVICE CONTROL METHOD, AND ELECTRONIC DEVICE CONTROL PROGRAM**

(57)   An electronic device includes a compression portion, a pressure regulation unit, a pressure sensor, and a control unit. The compression portion compresses a target region of a subject. The pressure regulation unit regulates an internal pressure of the compression portion. The pressure sensor detects the internal pressure of the compression portion. The control unit estimates a state of glucose metabolism or lipid metabolism of the subject on the basis of the internal pressure of the compression portion detected by the pressure sensor in a period during which the pressure regulation unit changes the internal pressure of the compression portion.

FIG. 1

EP 3 915 468 A1

**Description**

Cross Reference to Related Applications

The present application claims priority to Japanese Patent Application No. 2019-8542 filed in Japan on January 22, 2019 and Japanese Patent Application No. 2019-91652 filed in Japan on May 14, 2019, the entire disclosure of which is incorporated herein by reference.

Technical Field

The present disclosure relates to an electronic device, a method for controlling the electronic device, and a program for controlling the electronic device. More specifically, the present disclosure relates to an electronic device for estimating the health condition of a subject from measured biometric information, a method for controlling the electronic device, and a program for controlling the electronic device.

Background Art

In the related art, examples of means for estimating the health condition of a subject (user) include measuring blood components, and measuring blood fluidity. Such measurement can be performed by (invasively) using blood drawn from the subject. There is also known an electronic device that non-invasively measures biometric information from a target region of a subject, such as a wrist. For example, PTL 1 discloses an electronic device wearable on a wrist of a subject to measure the pulse of the subject.

Citation List

Patent Literature

PTL 1: Japanese Unexamined Patent Application Publication No. 2002-360530

Summary of Invention

An electronic device according to an embodiment includes a compression portion, a pressure regulation unit, a pressure sensor, and a control unit.
The compression portion compresses a target region of a subject.
The pressure regulation unit regulates an internal pressure of the compression portion.
The pressure sensor detects the internal pressure of the compression portion.
The control unit estimates a state of glucose metabolism or lipid metabolism of the subject on the basis of the internal pressure of the compression portion detected by the pressure sensor in a period during which the pressure regulation unit changes the internal pressure of the compression portion.
An electronic device according to an embodiment includes a compression portion, a pressure regulation unit, a pressure sensor, and a control unit.
The compression portion compresses a target region of a subject.
The pressure regulation unit regulates an internal pressure of the compression portion.
The pressure sensor detects the internal pressure of the compression portion.
The control unit estimates a state of glucose metabolism or lipid metabolism of the subject on the basis of the internal pressure of the compression portion detected by the pressure sensor in a period during which the pressure regulation unit maintains the internal pressure of the compression portion after the pressure regulation unit changes the internal pressure of the compression portion.
A method for controlling an electronic device according to an embodiment includes the following steps (1) to (4):

(1) a step of compressing a target region of a subject with a compression portion;
(2) a step of regulating an internal pressure of the compression portion with a pressure regulation unit;
(3) a step of detecting the internal pressure of the compression portion with a pressure sensor; and
(4) a step of estimating a state of glucose metabolism or lipid metabolism of the subject on the basis of the internal pressure of the compression portion detected by the pressure sensor in a period during which the pressure regulation unit changes the internal pressure of the compression portion.

A program for controlling an electronic device according to an embodiment causes a computer to execute the steps (1) to (4) above.

Brief Description of Drawings

[Fig. 1] Fig. 1 is a functional block diagram illustrating an example schematic configuration of an electronic device according to a first embodiment.
[Fig. 2] Fig. 2 is a graph illustrating an example of a change in the internal pressure of a cuff, which is detected by a pressure sensor, with respect to time.
[Fig. 3] Fig. 3 is a graph illustrating an example correction of a change in the internal pressure of the cuff with respect to time.
[Fig. 4] Fig. 4 is a graph illustrating an example of a change in the internal pressure of the cuff with respect to time, which is corrected using a digital filter.
[Fig. 5] Fig. 5 is a graph illustrating an example of a change in the internal pressure of the cuff with respect to time, which is corrected without using a digital filter.
[Fig. 6] Fig. 6 is a graph illustrating another example of a corrected change in the internal pressure of the cuff with respect to time.
[Fig. 7] Fig. 7 is a flowchart illustrating the operation of the electronic device according to the first embodiment.
[Fig. 8] Fig. 8 is a diagram illustrating an example estimation method based on a change in pulse wave using the electronic device according to the first embodiment.
[Fig. 9] Fig. 9 is a diagram illustrating an example of an acceleration pulse wave.
[Fig. 10] Fig. 10 is a diagram illustrating an example of acquired pulse waves.
[Fig. 11A] Fig. 11A is a diagram illustrating another example estimation method based on a change in pulse wave using the electronic device according to the first embodiment.
[Fig. 11B] Fig. 11B is a diagram illustrating the other example estimation method based on a change in pulse wave using the electronic device according to the first embodiment.
[Fig. 12] Fig. 12 is a flowchart for creating estimation formulas used by the electronic device according to the first embodiment.
[Fig. 13] Fig. 13 is a flowchart for estimating the blood glucose levels of the subject before and after a meal using the estimation formulas created through the flowchart illustrated in Fig. 12.
[Fig. 14] Fig. 14 is a diagram illustrating a comparison between blood glucose levels before and after a meal, which are estimated using the estimation formulas created through the flowchart illustrated in Fig. 12, and actually measured blood glucose levels before and after a meal.
[Fig. 15] Fig. 15 is a flowchart for creating estimation formulas according to another embodiment.
[Fig. 16] Fig. 16 is a flowchart for estimating the lipid value of the subject using the estimation formulas created through the flowchart illustrated in Fig. 14.
[Fig. 17] Fig. 17 is a graph illustrating an example of a change in the internal pressure of the cuff, which is detected by the pressure sensor, with respect to time.
[Fig. 18] Fig. 18 is a flowchart illustrating the operation of an electronic device according to a second embodiment.
[Fig. 19] Fig. 19 is a diagram illustrating an example of an acquired pulse wave.

Description of Embodiments

An electronic device capable of reducing the burden imposed on a subject for measuring biometric information of the subject can increase its usability. It is an object of the present disclosure to provide an electronic device with high usability, a method for controlling the electronic device, and a program for controlling the electronic device. According to an embodiment, it is possible to provide an electronic device with increased usability, a method for controlling the electronic device, and a program for controlling the electronic device. Some embodiments will be described in detail hereinafter with reference to the drawings.

(First Embodiment)

An electronic device according to a first embodiment can estimate the state of glucose metabolism, for example, the blood glucose level, of the subject or the state of lipid metabolism, for example, the lipid value, of the subject. The electronic device according to the first embodiment can have a configuration that is similar to the hardware of an existing oscillometric sphygmomanometer, for example. On the other hand, the electronic device according to the first embodiment performs operation different from that of the existing oscillometric sphygmomanometer. With this operation, the electronic device according to the first embodiment can obtain much information, such as glycolipid information, in addition to blood pressure information.

The configuration of the electronic device according to the first embodiment will be described hereinafter. As described above, the electronic device according to the first embodiment can have a configuration that is similar to the hardware of an existing oscillometric sphygmomanometer, for example. Thus, the description of a configuration similar to the configuration of the existing oscillometric sphygmomanometer will be simplified or omitted, as necessary. For example, in a configuration similar to that of the existing oscillometric sphygmomanometer, for example, a different algorithm or program (application software) is executed in parallel to the algorithm of the existing oscillometric sphygmomanometer, thus making it possible to implement the electronic device according to the first embodiment.

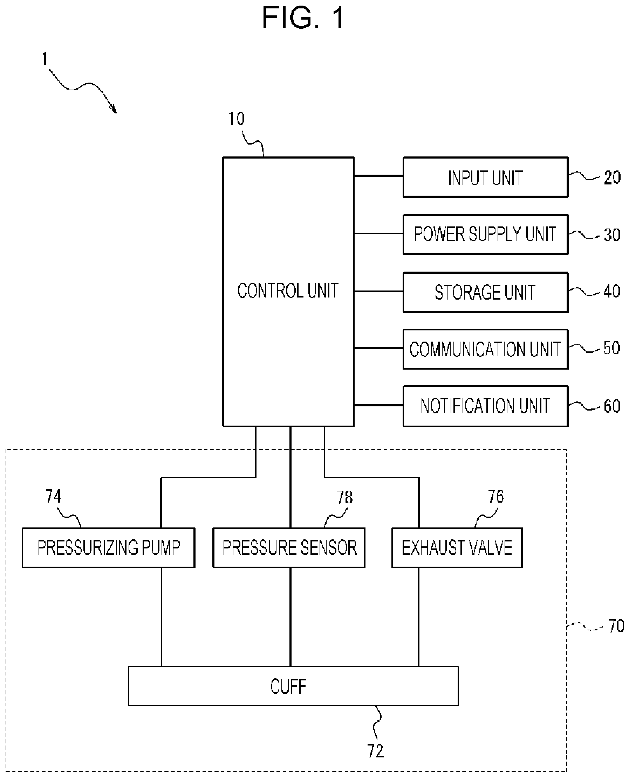

Fig. 1 is a functional block diagram of the electronic device according to the first embodiment. As illustrated in Fig. 1, an electronic device 1 according to the first embodiment includes a control unit 10, an input unit 20, a power supply unit 30, a storage unit 40, a communication unit 50, and a notification unit 60. In the first embodiment, not all of the control unit 10, the input unit 20, the power supply unit 30, the storage unit 40, the communication unit 50, and the notification unit 60 may be included in a housing of a single electronic device 1. In this case, the functional unit(s) not included in the housing of the electronic device 1 may be connected to the electronic device 1 in at least one of wired and wireless manners, as necessary.

The control unit 10 is a processor that controls and manages the entire electronic device 1, including the individual functional units of the electronic device 1. Further, the control unit 10 is a processor that performs, from the acquired information, processing and/or computation related to the estimation of the blood glucose level of the subject and the like. The control unit 10 is constituted by a processor, for example, a CPU (Central Processing Unit), which executes a program specifying a control procedure and a program for estimating the blood glucose level of the subject. These programs are stored in a storage medium such as the storage unit 40, for example. Further, the control unit 10 estimates a state related to glucose metabolism or lipid metabolism of the subject, and the like on the basis of the acquired information. The control unit 10 may cause the notification unit 60 to notify data.

The input unit 20 is configured to receive (detect) an operation input from the subject and is constituted by, for example, operation buttons (operation keys). The input unit 20 may be constituted by a touch screen, for example.

The power supply unit 30 includes, for example, a lithium-ion battery and a control circuit or the like for charging and discharging the lithium-ion battery, and supplies electric power to the entire electronic device 1. The power supply unit 30 is not limited to a secondary battery such as a lithium-ion battery and may be, for example, a primary battery such as a button battery. Alternatively, the power supply unit 30 may not be a primary battery or a secondary battery, but may be, for example, a functional unit that supplies electric power from the outside of the electronic device 1.

The storage unit 40 stores programs and data. The storage unit 40 may include a non-transitory storage medium such as a semiconductor storage medium and/or a magnetic storage medium. The storage unit 40 may include a plurality of types of storage media. The storage unit 40 may include a combination of a portable storage medium, such as a memory card, an optical disk, or a magneto-optical disk, and a storage medium reading device. The storage unit 40 may include a storage device used as a temporary storage area such as a RAM (Random Access Memory). The storage unit 40 stores various types of information and/or programs for operating the electronic device 1, and also functions as a work memory. The storage unit 40 may store, for example, information acquired by a blood pressure measurement unit 70 described below, and so on.

The communication unit 50 performs wired communication and/or wireless communication with an external device to transmit and receive various data. For example, the communication unit 50 communicates with an external device that stores biometric information of the subject to manage the health condition. The communication unit 50 transmits, to the external device, the result measured by the electronic device 1 and/or the health condition or the like estimated by the electronic device 1.

The notification unit 60 notifies the subject or the like of information by at least one of sound, vibration, an image, and so on. The notification unit 60 may include at least one of a speaker, a vibrator, and a display device. The display device may be, for example, a liquid crystal display (LCD: Liquid Crystal Display), an organic EL display (OELD: Organic Electro-Luminescence Display), an inorganic EL display (IELD: Inorganic Electro-Luminescence Display), or the like. In the first embodiment, the notification unit 60 may notify the subject or the like of, for example, the state of glucose metabolism or lipid metabolism and the like of the subject.

As illustrated in Fig. 1, the control unit 10 of the electronic device 1 according to the first embodiment is connected to the blood pressure measurement unit 70. The blood pressure measurement unit 70 can measure the blood pressure value of the subject. That is, the electronic device 1 according to the first embodiment may have a blood pressure value measurement function. In this case, the electronic device 1 illustrated in Fig. 1 may have, for example, a blood pressure value measurement function of an existing, so-called cuff-type sphygmomanometer. The blood pressure measurement unit 70 may constitute a portion of the electronic device 1 or may constitute a functional unit different from the electronic device 1.

As illustrated in Fig. 1, the blood pressure measurement unit 70 includes a cuff 72, a pressurizing pump 74, an exhaust valve 76, and a pressure sensor 78.

The cuff 72 can be wrapped around and worn on, for example, an arm (upper arm), a wrist, a finger, or the like including the target region of the subject. The cuff 72 may have a band shape of a predetermined width and includes an air bag into which air can be delivered. The cuff 72 compresses the target region of the subject by the pressure of the air supplied to the air bag. Accordingly, the cuff 72 according to the first embodiment may function as a compression portion in the present disclosure. The pressure of the air inside the air bag of the cuff 72 (or the compression portion) is hereinafter also referred to as the internal pressure of the cuff 72 (or the compression portion). In the first embodiment, the cuff 72 may be, for example, a cuff used in a typical cuff-type sphygmomanometer.

The pressurizing pump 74 is connected to the cuff 72 via an air tube. The pressurizing pump 74 can supply air to the air bag in a state in which the cuff 72 is wrapped around the arm, wrist, or finger of the subject. Accordingly, the pressurizing pump 74 can increase the internal pressure of the cuff 72. In response to air being supplied to the air bag, the cuff 72 tightens on the arm, wrist, or finger of the subject and compresses the blood vessels.

The exhaust valve 76 is connected to the cuff 72 via an air tube. The exhaust valve 76 discharges the air in the air bag of the cuff 72 to the outside. Accordingly, the exhaust valve 76 can reduce the internal pressure of the cuff 72.

The pressure sensor 78 detects the pressure in the air bag of the cuff 72. That is, that is, the pressure sensor 78 detects the internal pressure of the cuff 72. The pressure sensor 78 outputs a signal related to the pressure detected in the way described above to the control unit 10. The pressure sensor 78 may be disposed inside the cuff 72, for example.

In the first embodiment, a pressure regulation unit in the present disclosure may be configured to include at least one of the pressurizing pump 74 that increases the internal pressure of the cuff 72 and the exhaust valve 76 that reduces the internal pressure of the cuff 72. In this case, the pressure regulation unit in the present disclosure regulates the internal pressure of the cuff 72 (or the compression portion). The pressurizing pump 74 and the exhaust valve 76 may be controlled by the control unit 10 on the basis of the pressure in the air bag, which is acquired by the pressure sensor 78, for example. At least one of the pressurizing pump 74 and the exhaust valve 76 is hereinafter also referred to as the pressure regulation unit (74, 76), as necessary. The electronic device 1 can regulate the pressure in the air bag of the cuff 72 to measure the blood pressure value of the subject using a conventionally known method.

Next, the operation of the electronic device 1 according to the first embodiment will be described.

In the related art, there is known a method for measuring the blood pressure of the subject using an electronic device (for example, an oscillometric sphygmomanometer) having a configuration similar to that of the electronic device 1 according to the first embodiment (for example, the oscillometric method). In the related art, also, there is conceivable a method for measuring the blood pressure of the subject using an electronic device such as the electronic device 1 according to the first embodiment, followed by detection of a pulse wave based on the pulsation of the subject, to estimate the state of glucose metabolism or lipid metabolism of the subject (hereinafter also referred to simply as the "conceived method"). An overview of an operation implementing the conceived method described above using the electronic device 1 according to the first embodiment will be described hereinafter.

The conceived method may be started when, for example, the subject performs a predetermined input operation on the electronic device 1 while wearing the cuff 72 after a meal.

Upon receipt of the predetermined input operation by the subject, the electronic device 1 supplies air to the air bag of the cuff 72 through the pressurizing pump 74 to pressurize the arm, wrist, finger, or the like of the subject (hereinafter also referred to as the "first pressurization operation").

Then, the electronic device 1 exhausts the air in the air bag of the cuff 72 (for example, constant-rate exhaust) through the exhaust valve 76 to gradually depressurize the cuff 72 (hereinafter also referred to as the "depressurization operation"). During the depressurization operation, the pressure sensor 78 detects the internal pressure of the cuff 72. In this way, the electronic device 1 can acquire the blood pressure value of the subject after a meal, for example, using the conventionally known method.

Then, the electronic device 1 again supplies air to the air bag of the cuff 72 through the pressurizing pump 74 to pressurize the arm, wrist, finger, or the like of the subject (hereinafter also referred to as the "second pressurization operation"). The pressure at this time may be, for example, a predetermined pressure at which the electronic device 1 can acquire a pulse wave, and may be, for example, a pressure higher than the maximum blood pressure of the subject by a predetermined value (for example, 35 mmHg). This pressure may be a pressure at which a pulse wave can be stably acquired.

Then, the electronic device 1 holds the pressure of the cuff 72 constant and measures the pulse wave of the subject (hereinafter also referred to as the "holding operation"). That is, during this holding operation, the pressure sensor 78 detects the internal pressure of the cuff 72. In this way, the electronic device 1 can acquire, for example, the pulse wave of the subject after a meal on the basis of the internal pressure of the cuff 72. Then, the electronic device 1 can estimate, based on the pulse wave of the subject, the state of glucose metabolism or lipid metabolism of the subject using estimation formulas. The state of glucose metabolism or lipid metabolism of the subject can be, for example, the blood glucose level of the subject.

As described above, in the conceived method, the electronic device 1 according to the first embodiment can estimate the state of glucose metabolism or lipid metabolism of the subject through (1) the first pressurization operation, (2) the depressurization operation, (3) the second pressurization operation, and (4) the holding operation.

In the conceived method described above, however, pressurization is performed twice, as in (1) the first pressurization operation and (3) the second pressurization operation. In the conceived method, furthermore, the pressure sensor 78 detects the internal pressure of the cuff 72 twice, as in (2) the depressurization operation and (4) the holding operation. The reason for this is that, to determine the internal pressure of the cuff 72 to acquire the pulse wave of the subject, first, the blood pressure of the subject is measured.

In the conceived method, therefore, the time required to estimate the state of glucose metabolism or lipid metabolism of the subject is relatively long. In the conceived method, since the internal pressure of the cuff 72 in the pressurized state is detected twice, the physical and psychological burden on the subject, the efforts of detection, and the like are relatively large.

In view of such circumstances, the present disclosure proposes a method for reducing the burden imposed on the subject when the electronic device 1 according to the first embodiment estimates the state of glucose metabolism or lipid metabolism of the subject (hereinafter also referred to simply as the "proposed method"). According to the proposed method, it is possible to estimate the state of glucose metabolism or lipid metabolism of the subject without performing (3) the second pressurization operation and (4) the holding operation described above. The following describes the proposed method described above in more detail using the electronic device 1 according to the first embodiment.

Also in the proposed method, (1) the first pressurization operation and (2) the depressurization operation described above may be performed in a manner similar to that in the conceived method described above.

That is, the proposed method may be started when, for example, the subject performs a predetermined input operation on the electronic device 1 according to the first embodiment while wearing the cuff 72 after a meal.

In the proposed method, upon receipt (detection) of the predetermined input operation by the subject, the electronic device 1 according to the first embodiment supplies air to the air bag of the cuff 72 through the pressurizing pump 74 to pressurize the arm, wrist, finger, or the like of the subject to a predetermined pressure ((1) the first pressurization operation). That is, the electronic device 1 increases the internal pressure of the cuff 72 to a predetermined pressure using the pressurizing pump 74. In the electronic device 1, the control unit 10 may control the pressurizing pump 74 to increase the internal pressure of the cuff 72. The predetermined pressure may be, for example, a pressure larger than a pressure assumed to be the maximum blood pressure of the subject. The predetermined pressure may be stored in, for example, the storage unit 40 as a preset value or may be a value input by the subject through the input unit 20. The predetermined pressure may be, for example, a predetermined pressure at which the electronic device 1 can acquire a pulse wave, and may be, for example, a pressure higher than the maximum blood pressure of the subject by a predetermined value (for example, 35 mmHg). The predetermined pressure may be a pressure at which a pulse wave can be stably acquired.

Then, the electronic device 1 exhausts the air in the air bag of the cuff 72 (for example, constant-rate exhaust) through the exhaust valve 76 to gradually depressurize the cuff 72 ((2) the depressurization operation). In the electronic device 1, the control unit 10 may control the exhaust valve 76 to reduce the internal pressure of the cuff 72. In the electronic device 1, for example, the control unit 10 may determine, based on the information stored in the storage unit 40, the rate at which the exhaust valve 76 depressurizes the cuff 72. Also in the proposed method, as in the conceived method, the pressure sensor 78 detects the internal pressure of the cuff 72 during this depressurization operation. In the electronic device 1, the control unit 10 controls the pressure sensor 78 to detect the internal pressure of the cuff 72. The control unit 10 may perform control to store information on the internal pressure of the cuff 72 detected by the pressure sensor 78 in the storage unit 40.

In the proposed method, the state of glucose metabolism or lipid metabolism of the subject can be estimated through the operations described above. That is, in the proposed method, the electronic device 1 increases the internal pressure of the cuff 72 in (1) the first pressurization operation, and then estimates the state of glucose metabolism or lipid metabolism of the subject on the basis of the internal pressure of the cuff 72 detected by the pressure sensor 78 during (2) the depressurization operation. The electronic device 1 estimates (measures) the blood pressure value using the oscillometric method. Such estimation will further be described hereinafter.

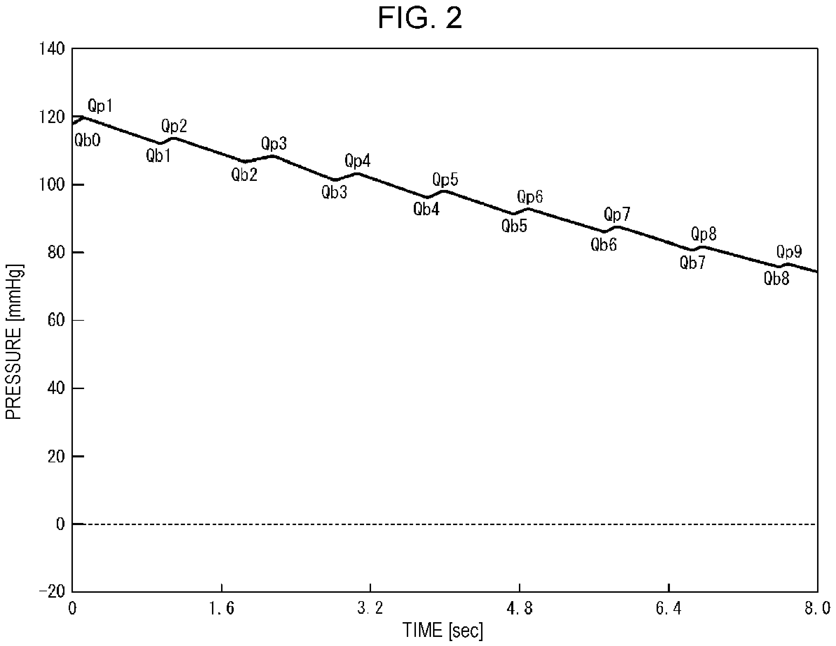

Fig. 2 is a graph illustrating an example of a change in the internal pressure of the cuff 72, which is detected by the pressure sensor 78 during (2) the depressurization operation described above, with respect to time. In Fig. 2, the horizontal axis represents elapsed time [seconds], and the vertical axis represents the pressure (the internal pressure of the cuff 72) [mmHg] detected by the pressure sensor 78.

As illustrated in Fig. 2, the internal pressure of the cuff 72 detected by the pressure sensor 78 repeatedly increases and decreases slightly over time due to pulsation of the subject. As illustrated in Fig. 2, the internal pressure of the cuff 72 detected by the pressure sensor 78 tends to gradually decrease as a whole because of the constant-rate exhaust by the exhaust valve 76.

When an increase in blood flow due to pulsation of the subject causes an expansion of the blood vessels, the internal pressure of the cuff 72 detected by the pressure sensor 78 increases. The pressure sensor 78 detects an expansion of the blood vessels caused by pulsation of the subject, such as peaks Qp1, Qp2, ..., and Qp9 illustrated in Fig. 2, as an increase in the internal pressure of the cuff 72 detected by the pressure sensor 78. In contrast, when a decrease in blood flow due to pulsation of the subject causes a contraction of the blood vessels, the internal pressure of the cuff 72 detected by the pressure sensor 78 slightly decreases. The pressure sensor 78 detects a contraction of the blood vessels caused by pulsation of the subject, such as bottoms Qb1, Qb2, ..., and Qb8 illustrated in Fig. 2, as a decrease in the internal pressure of the cuff 72 detected by the pressure sensor 78.

The graph illustrated in Fig. 2 illustrates a combination of the change in the internal pressure of the cuff 72 caused by the pulsation of the subject and the decrease in the internal pressure of the cuff 72 caused by the constant-rate exhaust of the exhaust valve 76. In the proposed method, accordingly, the control unit 10 corrects the curve, as illustrated in Fig. 2, indicating the change in the internal pressure of the cuff 72 with respect to time, in accordance with the influence of the reduction in the internal pressure of the cuff 72 caused by the constant-rate exhaust of the exhaust valve 76. For example, the control unit 10 may perform correction so that a pressure equal to the amount by which the internal pressure of the cuff 72 is reduced due to the constant-rate exhaust of the exhaust valve 76 is added to the internal pressure of the cuff 72.

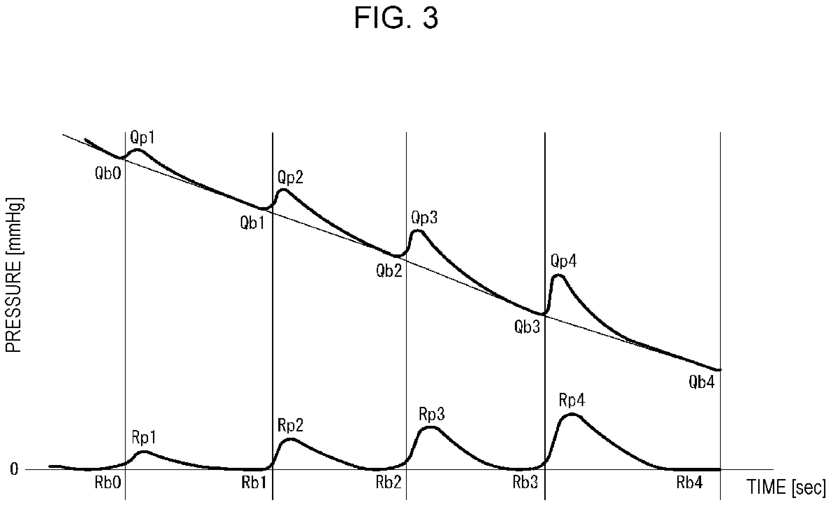

Fig. 3 is a graph illustrating an example correction of the change in the internal pressure of the cuff 72 with respect to time illustrated in Fig. 2. The curve depicted in the upper portion of Fig. 3 indicates the change in the internal pressure of the cuff 72 with respect to time before correction. That is, the curve depicted in the upper portion of Fig. 3 is the same as the curve indicating the change in the internal pressure of the cuff 72 with respect to time illustrated in Fig. 2. In Fig. 3, as an example, only a portion of the graph illustrated in Fig. 2 is illustrated in an enlarged manner. The curve depicted in the lower portion of Fig. 3 indicates a corrected change in the internal pressure of the cuff 72 with respect to time. Also in Fig. 3, the horizontal axis represents elapsed time [seconds], and the vertical axis represents the pressure (the internal pressure of the cuff 72) [mmHg] detected by the pressure sensor 78.

As illustrated in Fig. 3, the control unit 10 may perform correction so that, for example, the pressure corresponding to the amount by which the internal pressure of the cuff 72 is reduced due to the constant-rate exhaust of the exhaust valve 76 is added to the internal pressure of the cuff 72. As described above, the internal pressure of the cuff 72 detected by the pressure sensor 78 tends to gradually decrease as a whole due to the influence of the constant-rate exhaust by the exhaust valve 76. Considering only the action of constant-rate exhaust by the exhaust valve 76, it is expected that the internal pressure of the cuff 72 decreases substantially linearly or in a gentle curve over time. Accordingly, the control unit 10 may correct the internal pressure of the cuff 72, which gradually decreases due to the constant-rate exhaust of the exhaust valve 76, such that the internal pressure can be increased by the amount corresponding to the decrease.

In the example illustrated in Fig. 3, the bottoms Qb0, Qb1, Qb2, Qb3, and Qb4 indicated by the curve before the correction gradually decrease. Thus, in this case, the control unit 10 may approximate the change (decrease) in the values of the point Qb0, the point Qb1, the point Qb2, the point Qb3, the point Qb4, etc. with, for example, a straight line, line segments, a curve, or the like. For example, the control unit 10 may linearly approximate all of the point Qb0, the point Qb1, the point Qb2, the point Qb3, the point Qb4, etc. with a single straight line. Alternatively, the control unit 10 may approximate the interval between the point Qb0 and the point Qb1, the interval between the point Qb1 and the point Qb2, the interval between the point Qb2 and the point Qb3, the interval between the point Qb3 and the point Qb4, and the like with line segments. For example, the control unit 10 may perform curve approximation (curve fitting) on the point Qb0, the point Qb1, the point Qb2, the point Qb3, the point Qb4, etc. The control unit 10 may perform correction such that the change in pressure with respect to time (decrease over time), which is obtained by the approximation described above, is added to the value of the internal pressure of the cuff 72 detected by the pressure sensor 78. As a result, the values of the bottoms Qb0, Qb1, Qb2, Qb3, Qb4, etc. become the same (or substantially the same) value after the correction.

In Fig. 3, the bottoms Qb0, Qb1, Qb2, Qb3, and Qb4 of the curve before the correction are represented as bottoms Rb0, Rb1, Rb2, Rb3, and Rb4 of a curve after the correction, respectively. The bottoms Rb0, Rb1, Rb2, Rb3, and Rb4 of the curve after the correction depicted in the lower portion of Fig. 3 have the same (or substantially the same) value. Fig. 3 illustrates further correction such that the value of the bottom Qb0 of the curve before the correction depicted in the upper portion becomes the bottom Rb0 (zero) of the curve after the correction depicted in the lower portion. That is, Fig. 3 illustrates that the curve before the correction (upper portion) is corrected in accordance with depressurization performed by the exhaust valve 76 through the constant-rate exhaust and that further correction is performed to make the bottoms of the curve equal to (substantially) zero to obtain the curve after the correction (lower portion).

In this way, in the proposed method, the control unit 10 may correct the change in the internal pressure of the cuff 72 detected by the pressure sensor 78 in accordance with depressurization performed by the pressure regulation unit (74, 76). Through the correction described above, the electronic device 1 according to the first embodiment can obtain a pulse wave caused by pulsation of the subject, such as the curve after the correction depicted in the lower portion of Fig. 3. The electronic device 1 according to the first embodiment may estimate the state of glucose metabolism or lipid metabolism of the subject on the basis of the pulse wave of the subject obtained in the way described above.

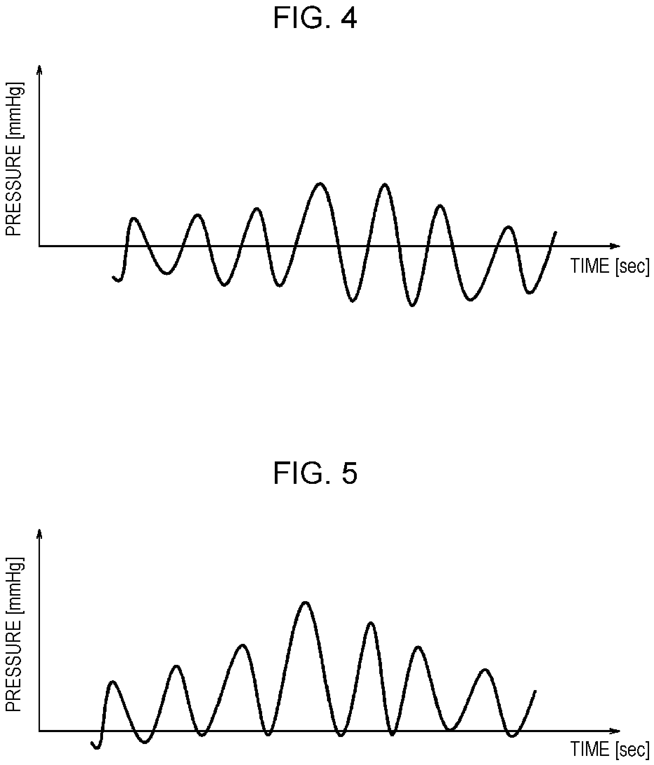

The correction described above can be performed through, for example, computation performed by the control unit 10 in the electronic device 1. In some cases, for example, analysis of a pulse wave using an existing sphygmomanometer such as of an oscillometric type may involve computational processing using a digital filter. However, analysis of a pulse wave using a digital filter in the proposed method may result in a waveform including an AC component, in which the internal pressure of the cuff 72 fluctuates in both the positive direction and the negative direction. For example, when the curve depicted in the upper portion of Fig. 3 is subjected to correction processing using a digital filter of an existing sphygmomanometer such as of an oscillometric type, a pulse wave as illustrated in Fig. 4 may be obtained. A curve illustrated in Fig. 4 includes an AC component because processing using a digital filter is performed. In such a waveform including an AC component, for example, low-frequency components around 1 Hz may be lost. In the waveform including an AC component, furthermore, the value of AI described below may change because of the loss of a feature of the pulse wave.

In the proposed method, accordingly, the control unit 10 performs computational processing without using a digital filter to analyze a pulse wave. As a result of the correction processing of the curve depicted in the upper portion of Fig. 3 without using a digital filter, for example, a pulse wave as illustrated in Fig. 5 is obtained. A curve illustrated in Fig. 5 does not include an AC component because processing is performed without using a digital filter. As illustrated in Fig. 5, analysis of a pulse wave without using a digital filter results in a waveform that does not include an AC component, in which the internal pressure of the cuff 72 hardly fluctuates in the negative direction. In this manner, when processing is performed without using a digital filter, the value of AI described below can be obtained in the proposed method without the loss of a feature of the pulse wave.

In this way, in the proposed method, the control unit 10 may correct the change in the internal pressure of the cuff 72 detected by the pressure sensor 78 without using a digital filter. Through the correction described above, the electronic device 1 according to the first embodiment can obtain the pulse wave of the subject, such as the curve after the correction depicted in the lower portion of Fig. 3. The electronic device 1 according to the first embodiment may estimate the state of glucose metabolism or lipid metabolism of the subject on the basis of the pulse wave of the subject obtained in the way described above.

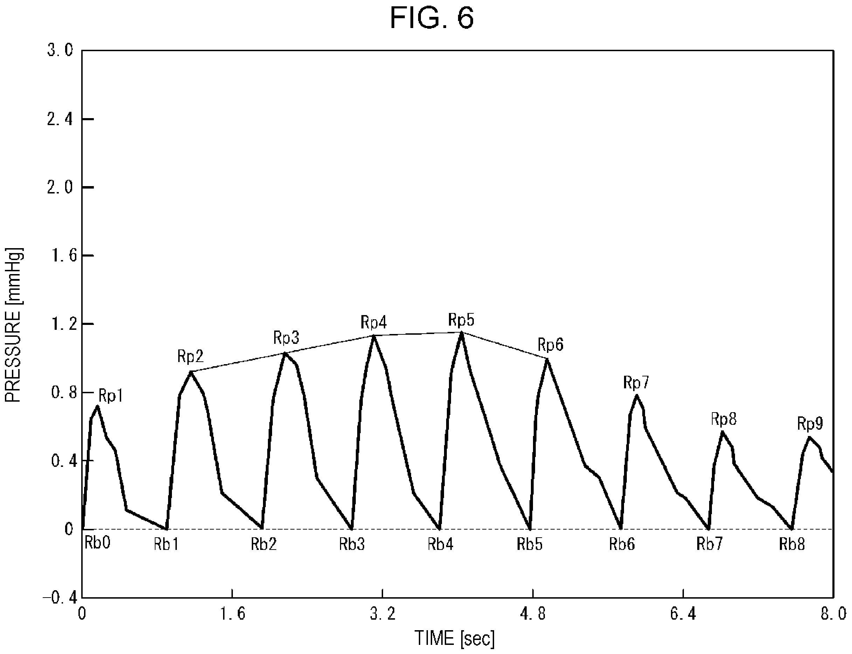

In the curve after the correction depicted in the lower portion of Fig. 3, it is not always easy to compare the magnitudes of a plurality of pulse waves, each pulse wave having one wavelength, in the pressure direction (for example, the respective magnitudes of peaks Rp1, Rp2, Rp3, and Rp4). An enlarged version of the curve after the correction depicted in the lower portion of Fig. 3 in the pressure direction is illustrated in Fig. 6.

Fig. 6 is a diagram illustrating the curve after the correction depicted in the lower portion of Fig. 3 in an enlarged manner in the vertical direction (pressure-axis direction). As described above, Fig. 3 illustrates only a portion of the graph illustrated in Fig. 2 in an enlarged manner. In Fig. 6, in contrast, the horizontal direction (time-axis direction) is illustrated so as to correspond to that in Fig. 2, and the vertical direction (pressure-axis direction) is illustrated in a more enlarged manner than that in Fig. 2.

In the proposed method, when the pulse wave indicated by the curve after the correction as illustrated in Fig. 6 is regarded as a plurality of pulse waves each having one wavelength, the control unit 10 may estimate the state of glucose metabolism or lipid metabolism of the subject on the basis of at least one pulse wave among the plurality of pulse waves. For example, the control unit 10 may estimate the state of glucose metabolism or lipid metabolism of the subject on the basis of at least one of the pulse wave from the point Rb0 to the point Rb1, the pulse wave from the point Rb1 to the point Rb2, the pulse wave from the point Rb2 to the point Rb3, ..., and the pulse wave from the point Rb7 to the point Rb8. In this case, the control unit 10 may estimate a plurality of glucose metabolisms or lipid metabolisms of the subject on the basis of the average or the like of the plurality of pulse waves. Alternatively, the control unit 10 may estimate a plurality of states of glucose metabolism or lipid metabolism of the subject on the basis of the plurality of respective pulse waves.

In the example illustrated in Fig. 6, when the curve after the correction is regarded as a plurality of pulse waves, each pulse wave having one wavelength, the peak Rp5 is largest among the peaks (Rp1 to Rp9) of the plurality of pulse waves. In this case, the control unit 10 may estimate the state of glucose metabolism or lipid metabolism of the subject on the basis of a pulse wave including the largest peak Rp5. In the pulse wave after the correction illustrated in Fig. 6, when the internal pressure of the cuff 72 reaches a peak, the pulsation of the subject has the largest amplitude. When the internal pressure of the cuff 72 becomes the average blood pressure, the blood vessels in the target region of the subject are brought into a state close to an unloaded state. The blood vessels are most freely movable in the unloaded state. In the blood vessels in the unloaded state, therefore, the amplitude caused by pulsation is very large. It is considered that the blood pressure value of the subject is the average (or close to the average) at the largest peak in the plurality of pulse waves in the curve after the correction, such as the peak Rp5 illustrated in Fig. 6.

As described above, the pulse wave greatly changes depending on the relationship between the cuff pressure and the blood pressure. In this method using the features of the pulse wave, it is very important to determine the cuff pressure at which the pulse wave is measured. In the pulse wave after the correction as illustrated in Fig. 6, the pulse wave at the average blood pressure is based on the pulse wave including the largest peak, and the pulse wave amplitude is the largest, resulting in a condition with a good SN (signal-to-noise ratio). According to this, a good estimation result can be obtained by estimating the state of glucose metabolism or lipid metabolism of the subject.

In this way, in the proposed method, the control unit 10 may estimate the state of glucose metabolism or lipid metabolism of the subject on the basis of the pulse wave having the largest peak among the pulse waves of the subject.

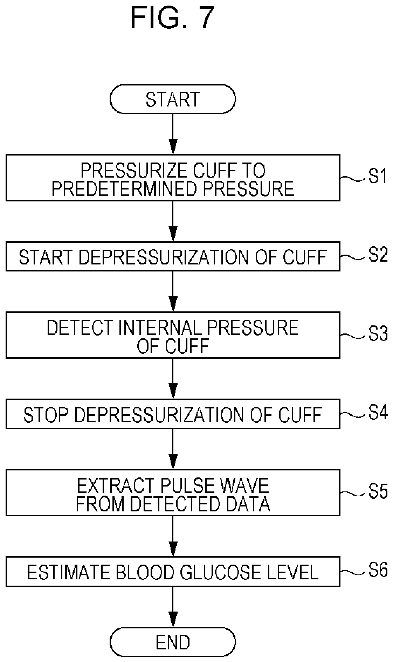

Next, the operation of the electronic device 1 in the proposed method described above will be described. Fig. 7 is a flowchart illustrating an operation of estimating the state of glucose metabolism or lipid metabolism of the subject using the electronic device 1 according to the first embodiment.

When the operation illustrated in Fig. 7 starts, the control unit 10 controls the pressurizing pump 74 to increase the internal pressure of the cuff 72 to a predetermined pressure (step S1). The operation of step S1 corresponds to (1) the first pressurization operation described above.

When the internal pressure of the cuff 72 is increased in step S1, the control unit 10 controls the exhaust valve 76 to start reducing the internal pressure (for example, constant-rate depressurization) of the cuff 72 (step S2).

When the internal pressure of the cuff 72 is started to be reduced in step S2, the control unit 10 detects the internal pressure of the cuff 72 using the pressure sensor 78 (step S3).

When the detection of the internal pressure of the cuff 72 in step S3 is completed, the control unit 10 controls the exhaust valve 76 to stop reducing the internal pressure (for example, constant-rate depressurization) of the cuff 72 (step S4). The trigger upon which the process proceeds from step S3 to step S4 may be, for example, the point in time at which a predetermined time period has elapsed. The trigger upon which the process proceeds from step S3 to step S4 may be, for example, the point in time at which the internal pressure of the cuff 72 detected by the pressure sensor 78 has reached a predetermined pressure. The trigger upon which the process proceeds from step S3 to step S4 may be, for example, the point in time at which at least one pulse wave has been detected a predetermined number of times. The operation of steps S1 to S4 corresponds to (2) the depressurization operation described above. At the point in time at which the operation up to step S4 is completed, the control unit 10 can obtain, for example, the change in the internal pressure of the cuff 72 with respect to time as illustrated in Fig. 2.

When the reduction in the internal pressure of the cuff 72 is stopped in step S4, the control unit 10 extracts a pulse wave on the basis of the change in the internal pressure of the cuff 72 detected in step S3 with respect to time (step S5). In step S5, the control unit 10 extracts, for example, the pulse wave as illustrated in Fig. 6 from, for example, the change in the internal pressure of the cuff 72 with respect to time as illustrated in Fig. 2.

When the pulse wave is extracted in step S5, the control unit 10 estimates, for example, the glucose metabolism of the subject, such as the blood glucose level, on the basis of the extracted pulse wave (step S7). In step S7, the control unit 10 may estimate, for example, the lipid metabolism of the subject, such as the lipid value, instead of the glucose metabolism of the subject or together with the glucose metabolism of the subject. The method for estimating the blood glucose level or the like on the basis of a pulse wave, as performed in step S7, will further be described below.

In this way, in the proposed method, the control unit 10 causes the pressure regulation unit (74, 76) to increase the internal pressure of the cuff 72 and then causes the pressure regulation unit (74, 76) to reduce the internal pressure of the cuff 72. Further, the control unit 10 causes the pressure sensor 78 to detect the internal pressure of the cuff 72 in a period during which the internal pressure of the cuff 72 is reduced after the internal pressure of the cuff 72 is increased. Then, the control unit 10 estimates the state of glucose metabolism or lipid metabolism of the subject on the basis of the internal pressure of the cuff 72 detected by the pressure sensor 78. The control unit 10 may estimate a blood glucose level as the glucose metabolism of the subject, or may estimate a lipid value as the lipid metabolism of the subject.

According to the proposed method, therefore, since only (1) the first pressurization operation and (2) the depressurization operation described above are performed, the time required to estimate the state of glucose metabolism or lipid metabolism of the subject is relatively short. In the proposed method, furthermore, since the internal pressure of the cuff 72 is detected only once in the pressurized state, the physical and psychological burden on the subject, the efforts of detection, and the like are also relatively small. That is, according to the proposed method for the electronic device 1 according to the first embodiment, the time required to estimate the state of glucose metabolism or lipid metabolism of the subject is shortened, and the burden imposed on the subject is also reduced. The proposed method can thus increase the usability of the electronic device 1 according to the first embodiment.

As described above, the electronic device 1 can acquire the blood pressure value of the subject using a conventionally known method, for example. Accordingly, in addition to performing the operation described above, the control unit 10 may further determine the blood pressure value of the subject on the basis of the internal pressure of the cuff 72 detected by the pressure sensor 78, and estimate the state of glucose metabolism or lipid metabolism of the subject on the basis of the blood pressure value.

Next, a method for estimating the blood glucose level or the like on the basis of the pulse wave as described with reference to Fig. 6 will be described.

The electronic device 1 according to the first embodiment may estimate the state of glucose metabolism. In the first embodiment, the electronic device 1 may estimate a blood glucose level as the state of glucose metabolism.

The electronic device 1 can estimate the blood glucose level of the subject on the basis of estimation formulas created through regression analysis. The electronic device 1 may store estimation formulas for estimating the blood glucose level on the basis of the pulse wave and the blood pressure value in, for example, the storage unit 40 or the like in advance. The electronic device 1 estimates the blood glucose level using these estimation formulas. In the following, the blood pressure value is a numerical value related to the blood pressure of the subject, and may include, for example, a maximum blood pressure, a minimum blood pressure, or a pulse pressure. The pulse pressure is the difference between the systolic blood pressure (maximum blood pressure) and the diastolic blood pressure (minimum blood pressure).

The theory related to the estimation of the blood glucose level based on a pulse wave will now be described. The blood glucose level in the blood increases after a meal, causing a reduction in blood fluidity (increase in viscosity), dilation of blood vessels, and an increase in the amount of circulating blood, and vascular dynamics and hemodynamics are determined so as to balance these states. The reduction in blood fluidity is caused by, for example, an increase in the viscosity of blood plasma or a decrease in the deformability of red blood cells. The dilation of blood vessels is caused by secretion of insulin, secretion of digestive hormones, a rise in body temperature, and so on. When blood vessels dilate, the blood pressure decreases, and the pulse pressure also changes. Then, the pulse rate increases to suppress the decrease in blood pressure. The increase in the amount of circulating blood compensates for blood consumption for digestion and absorption. Changes in vascular dynamics and hemodynamics between before and after a meal due to these factors are also reflected in the pulse wave. As described above, the blood pressure value and the pulse wave change between before and after a meal. Accordingly, the electronic device 1 can acquire blood pressure values and pulse waves before and after a meal and estimate the blood glucose level on the basis of the acquired changes in blood pressure value and pulse wave.

On the basis of the estimation theory described above, estimation formulas for estimating the blood glucose level can be created by performing regression analysis on the basis of sample data of blood pressure values, blood glucose levels, and pulse waves before and after a meal, which is obtained from a plurality of subjects. At the time of estimation, the created estimation formulas are applied to the index based on the pulse wave of the subject. Thus, the blood glucose level of the subject can be estimated. In the creation of estimation formulas, in particular, estimation formulas are created by performing regression analysis using sample data for which variations in blood glucose level are close to a normal distribution, thereby making it possible to estimate the blood glucose level of the subject being examined, regardless of before or after a meal.

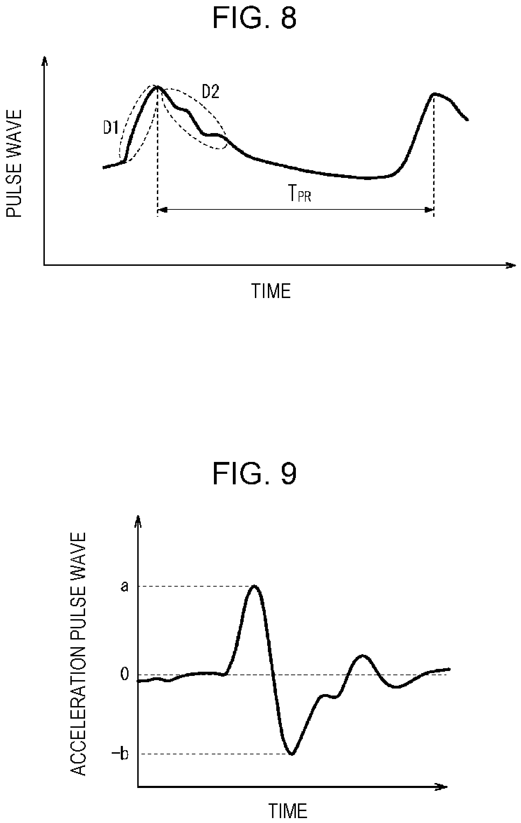

Fig. 8 is a diagram illustrating an example estimation method based on a change in pulse wave, and illustrates an example of a pulse wave. The estimation formulas for estimating the blood glucose level are created using regression analysis with an explanatory variable including the index based on the pulse wave. The index based on the pulse wave includes, for example, an index indicating the rising of a pulse wave (rising index) SI, the AI (Augmentation Index), and the pulse rate PR.

The rising index SI is derived based on a waveform indicated in an area D1 in Fig. 8. Specifically, the rising index SI is the ratio of the first local minimum value to the first local maximum value in an acceleration pulse wave derived by differentiating the pulse wave twice. The rising index SI is expressed by, for example, -b/a in the acceleration pulse wave illustrated as an example in Fig. 9. The rising index SI decreases because of a reduction in blood fluidity, secretion of insulin, dilation (relaxation) of blood vessels caused by a rise in body temperature, and so on after a meal.

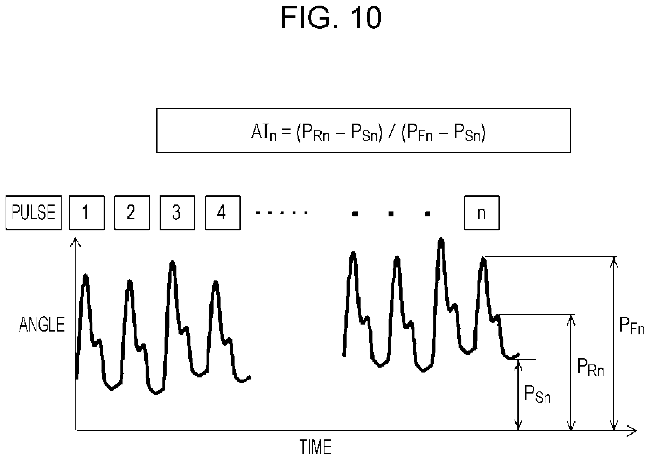

The AI is an index represented by the ratio of the magnitudes of the forward wave and the reflected wave of the pulse wave. A method for deriving the AI will be described with reference to Fig. 10. Fig. 10 is a diagram illustrating an example of pulse waves acquired using the electronic device 1. Fig. 10 illustrates a case where an angular velocity sensor is used as a means for sensing pulsation. However, the pulse wave as illustrated in Fig. 6 in the proposed method described above can also be considered in a similar manner. For example, the pulse waves in Fig. 10 may be considered as pulse waves obtained when the cuff pressure in Fig. 6 is clamped at the average blood pressure. In the disclosed example described above, the cuff pressure is exhausted at a constant rate (constant-rate exhaust), and the exhaust and depressurization have the same meaning. Accordingly, clamping means stopping the exhaust (depressurization). According to the first embodiment, the exhaust is stopped (= clamped) at the average blood pressure, thereby making it possible to measure the pulse wave in the maximum amplitude state. Fig. 10 illustrates an integration of the angular velocity acquired by the angular velocity sensor, with the horizontal axis representing time and the vertical axis representing the angle. The acquired pulse waves may contain noise caused by, for example, body movement of the subject and may thus be corrected by a filter that removes the DC (Direct Current) component to extract only the pulsation component.

The propagation of a pulse wave is a phenomenon in which a heartbeat caused by blood pumped out of the heart is transmitted through the wall of an artery or the blood. The heartbeat caused by blood pumped out of the heart reaches the peripheries of limbs as a forward wave, and a portion thereof is reflected at locations such as a blood vessel branch portion or a blood-vessel-diameter changing portion and returns as a reflected wave. The AI is obtained by dividing the magnitude of the reflected wave by the magnitude of the forward wave, and is expressed by $AI_n = (P_{Rn} - P_{Sn}) / (P_{Fn} - P_{sn})$. Here, $AI_n$ is the AI for each pulse. The AI may be obtained by, for example, measuring a pulse wave for several seconds and calculating an average value $AI_{ave}$ of $AI_n$ (n is an integer of 1 to n) for the respective pulses. The AI is derived based on a waveform indicated by an area D2 in Fig. 8. The AI decreases due to a reduction in blood fluidity, dilation of blood vessels due to a rise in body temperature, and so on after a meal.

The pulse rate PR is derived based on a period $T_{PR}$ of the pulse wave illustrated in Fig. 8. The pulse rate PR increases after a meal.

The electronic device 1 can estimate the blood glucose level using estimation formulas created based on the age, the rising index SI, the AI, the pulse rate PR, and the blood pressure value measured using a sphygmomanometer. The sphygmomanometer may be implemented using any sphygmomanometer, and examples thereof include a sphygmomanometer based on the oscillometric method and a sphygmomanometer based on the Riva-Rocci/Korotkoff method.

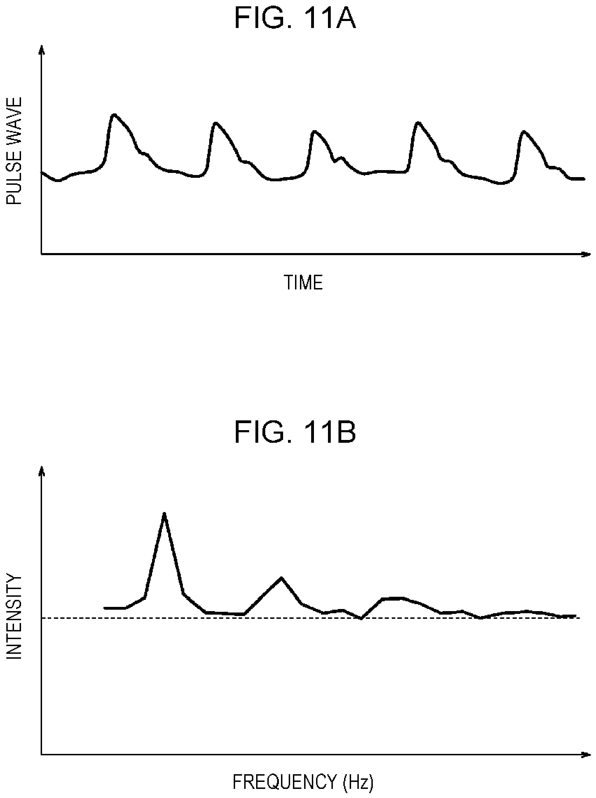

Fig. 11A and Fig. 11B are diagrams illustrating another example estimation method based on a change in pulse wave. Fig. 11A illustrates a pulse wave, and Fig. 11B illustrates the result of performing a fast Fourier transform (FFT) on the pulse wave in Fig. 11A. The estimation formulas for estimating the blood glucose level are created by, for example, regression analysis related to fundamental and harmonic components (Fourier coefficients) derived by the FFT. A peak value in the result of the FFT illustrated in Fig. 11B changes in accordance with a change in the waveform of the pulse wave. Therefore, the blood glucose level can be estimated using estimation formulas created based on the Fourier coefficients.

The electronic device 1 estimates the blood glucose level of the subject on the basis of the rising index SI, the AI, the pulse rate PR, and the pulse pressure described above, the Fourier coefficients, and so on using the estimation formulas.

A method for creating the estimation formulas used when the electronic device 1 estimates the blood glucose level of the subject will be described. The estimation formulas may be created by the electronic device 1 or may be created in advance using another computer or the like. A device that creates the estimation formulas is referred to as an estimation formula creation device and will be described hereinafter. The created estimation formulas are stored in advance in, for example, the storage unit 40 before the subject estimates the blood glucose level with the electronic device 1.

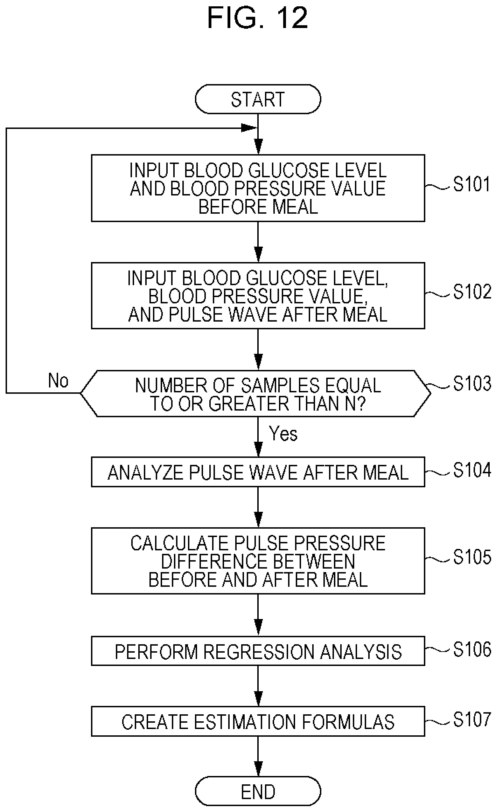

Fig. 12 is a flowchart for creating the estimation formulas used by the electronic device 1. The estimation formulas are created by measuring the blood glucose levels of the subject before and after a meal using a blood glucose meter, measuring the blood pressure value of the subject using a sphygmomanometer, measuring the pulse wave of the subject after the meal using a pulse wave meter, and performing regression analysis on the basis of sample data acquired through the measurement. The term before a meal refers to a time period during which the subject is fasting, and the term after a meal refers to a time period during which the blood glucose level rises after a predetermined time period elapses after a meal (for example, about one hour after the start of the meal). The acquired sample data is not limited to that before and after a meal, and is desirably data in a time slot in which the blood glucose level greatly varies.

In the creation of the estimation formulas, first, the blood glucose level and the blood pressure value of the subject before a meal, which are measured with a blood glucose meter and a sphygmomanometer, respectively, are input to the estimation formula creation device (step S101).

Further, information related to the blood glucose level, the blood pressure value, and the pulse wave associated with the blood glucose level of the subject after the meal, which are measured with the blood glucose meter, the sphygmomanometer, and a pulse wave meter, respectively, is input to the estimation formula creation device (step S102). The blood glucose levels input in step S101 and step S102 are measured with the blood glucose meter by, for example, drawing blood. In step S101 or step S102, the age of the subject in each piece of sample data is also input.

The estimation formula creation device determines whether the number of samples in the sample data input in step S101 and step S102 is equal to or greater than N, which is sufficient to perform regression analysis (step S103). The number of samples N can be determined as necessary and can be set to, for example, 100. If it is determined that the number of samples is less than N (in the case of No), the estimation formula creation device repeatedly performs step S101 and step S102 until the number of samples becomes equal to or greater than N. On the other hand, if it is determined that the number of samples becomes equal to or greater than N (in the case of Yes), the estimation formula creation device proceeds to step S104 and calculates the estimation formulas.

In the calculation of the estimation formulas, the estimation formula creation device analyzes the input pulse wave after the meal (step S104). In the first embodiment, the estimation formula creation device analyzes the rising index SI, the AI, and the pulse rate PR for the pulse wave after the meal. The estimation formula creation device may perform FFT analysis as the analysis of the pulse wave.

Further, the estimation formula creation device calculates the pulse pressures before and after the meal on the basis of the input blood pressure values before and after the meal, and calculates the difference (pulse pressure difference) DP between the pulse pressure before the meal and the pulse pressure after the meal (step S105).

Then, the estimation formula creation device performs regression analysis (step S106). The objective variable in the regression analysis is blood glucose levels before and after a meal. The explanatory variable in the regression analysis is the age input in step S101 or step S102, the rising index SI, the AI, and the pulse rate PR for the pulse wave after the meal analyzed in step S104, and the pulse pressure difference DP calculated in step S105. When the estimation formula creation device performs FFT analysis in step S104, the explanatory variable may be, for example, the Fourier coefficients calculated as a result of the FFT analysis.

The estimation formula creation device creates, based on the result of the regression analysis, estimation formulas for estimating the blood glucose levels before and after the meal (step S106). An example of the estimation formulas for estimating the blood glucose levels before and after the meal is given by Equations (1) and (2) below.

$$GLa = 1151.9 + 2.79 \times age + 5.27 \times DP - 0.25 \times PRa - 3.69 \times AIa + 6.07 \times Sla \quad (1)$$

$$GLb = 52.7 + 1.75 \times age + 3.28 \times DP + 2.52 \times PRa - 2.59 \times AIa + 1.03 \times Sla \quad (2)$$

In Equations (1) and (2), GLa and GLb represent blood glucose levels after and before a meal, respectively. Further, age represents the age of the subject, PRa represents the pulse rate PR after the meal, AIa represents the AI after the meal, and Sla represents the rising index SI after the meal.

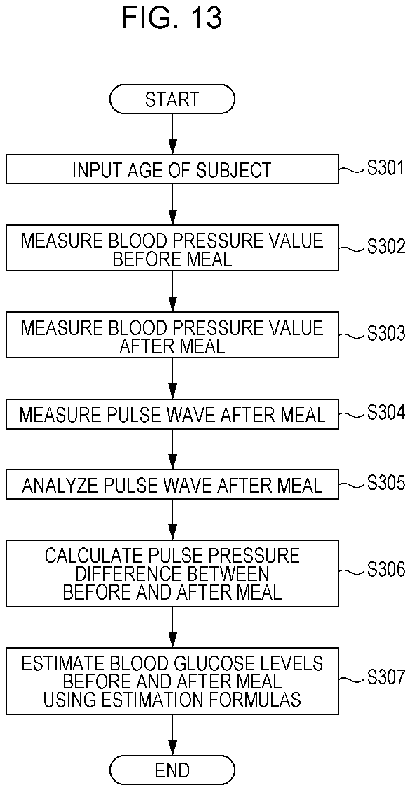

Next, a flowchart for estimating the blood glucose level of the subject using the estimation formulas will be described. Fig. 13 is a flowchart for estimating the blood glucose levels of the subject before and after a meal using the estimation formulas created through the flowchart illustrated in Fig. 12. Here, the flowchart executed by an electronic device 1 having a blood pressure value measurement function, such as the electronic device 1 according to the first embodiment, will be described.

First, the age of the subject is input to the electronic device 1 in response to an operation of the input unit 20 by the subject (step S301).

Further, the electronic device 1 measures the blood pressure value of the subject before a meal in response to an operation of the input unit 20 by the subject (step S302) .

Then, after the subject has eaten a meal, the electronic device 1 measures the blood pressure value of the subject after the meal in response to an operation of the input unit 20 by the subject (step S303).

Further, the electronic device 1 measures the pulse wave of the subject after the meal in response to an operation by the subject (step S304).

Then, the electronic device 1 analyzes the measured pulse wave (step S305). Specifically, the electronic device 1 analyzes, for example, the rising index SI, the AI, and the pulse rate PR related to the measured pulse wave.

Further, the electronic device 1 calculates the pulse pressures before and after the meal on the basis of the measured blood pressure values before and after the meal, and calculates the pulse pressure difference DP between before and after the meal (step S306).

The electronic device 1 applies the rising index SI, the AI, and the pulse rate PR analyzed in step S305, the pulse pressure difference DP between before and after the meal, which is calculated in step S306, and the age of the subject to, for example, Equations (1) and (2) described above to estimate the blood glucose levels of the subject before and after the meal (step S307). The subject is notified of the estimated blood glucose levels before and after the meal by, for example, the notification unit 60 of the electronic device 1.

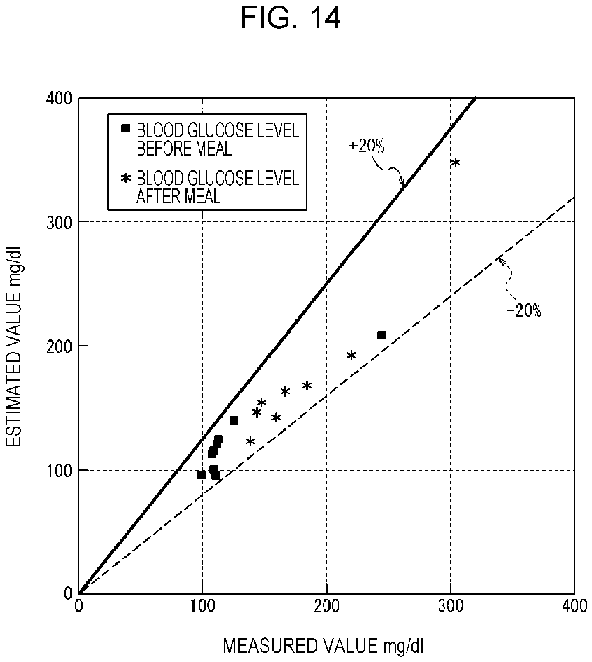

Fig. 14 is a diagram illustrating a comparison between the blood glucose levels before and after a meal, which are estimated using the estimation formulas created through the flowchart illustrated in Fig. 12, and the actually measured blood glucose levels before and after a meal. In the graph illustrated in Fig. 14, the horizontal axis represents the measured values (actually measured values) of the blood glucose level before and after a meal, and the vertical axis represents the estimated values of the blood glucose level before and after a meal. The measured values of the blood glucose level were measured using the blood glucose meter, Medisafe Fit, manufactured by Terumo Corporation. As illustrated in Fig. 14, the measured values and the estimated values are included in a range of substantially ±20%. That is, the estimation accuracy using the estimation formulas can be within 20%.

In this way, the electronic device 1 can estimate the blood glucose levels before and after a meal in a non-invasive manner and in a short time on the basis of the blood pressure values before and after the meal, which are measured by the subject using a sphygmomanometer. In particular, the AI is a parameter that can depend on the blood pressure value, and, as with the electronic device 1, the blood glucose level is estimated based on estimation formulas created such that the blood pressure value is included as an explanatory variable. Thus, the estimation accuracy of the blood glucose level can be improved. In the first embodiment, estimation formulas are created using the blood glucose levels before and after a meal, the blood pressure values before and after the meal, and the pulse wave after the meal.

However, the creation of the estimation formulas is not limited to this, and estimation formulas may be created using the blood glucose level after the meal, and the blood pressure value and the pulse wave either before or after the meal. Further, the electronic device 1 may estimate the blood glucose level of the subject at any timing, instead of estimating the blood glucose levels before and after a meal. The electronic device 1 can also estimate the blood glucose level at any timing in a non-invasive manner and in a short time.

The electronic device 1 according to the first embodiment may update the estimation formulas stored in the storage unit 40 on the basis of the blood pressure values of the subject before and after the meal, which are acquired in step S302 and step S303 during the estimation of the blood glucose level. That is, the electronic device 1 can use the blood pressure values before and after the meal and the pulse wave after the meal, which are acquired for the estimation of the blood glucose level, as sample data for updating the estimation formulas. Accordingly, the estimation formulas are updated each time the subject estimates a blood glucose level, and the estimation accuracy of the blood glucose levels before and after a meal using the estimation formulas is improved.

(Other Embodiment)

In the first embodiment described above, a case has been described in which the electronic device 1 estimates the blood glucose levels of the subject before and after a meal. Next, another embodiment, that is, an example in which the electronic device 1 estimates the state of lipid metabolism of the subject, will be described. In another embodiment, the electronic device 1 estimates the lipid value after a meal as the state of lipid metabolism. The lipid value includes triglycerides, total cholesterol, HDL cholesterol, LDL cholesterol, and the like. In the description of another embodiment, points similar to those of the first embodiment described above will not be described, as necessary.

The electronic device 1 stores estimation formulas for estimating a lipid value on the basis of a pulse wave in the storage unit 40 in advance, for example. The electronic device 1 estimates the lipid value using these estimation formulas.

The estimation theory related to the estimation of a lipid value based on a pulse wave is similar to the estimation theory for a blood glucose level described in the first embodiment. That is, a change in the lipid value in the blood is also reflected in a change in the waveform of the pulse wave and a change in blood pressure value. Accordingly, the electronic device 1 can acquire a blood pressure value and a pulse wave and estimate the lipid value on the basis of a change in the acquired blood pressure value and pulse wave. The electronic device 1 estimates the lipid value using a pulse wave and a blood pressure value during lipid estimation to improve the estimation accuracy of the lipid value.

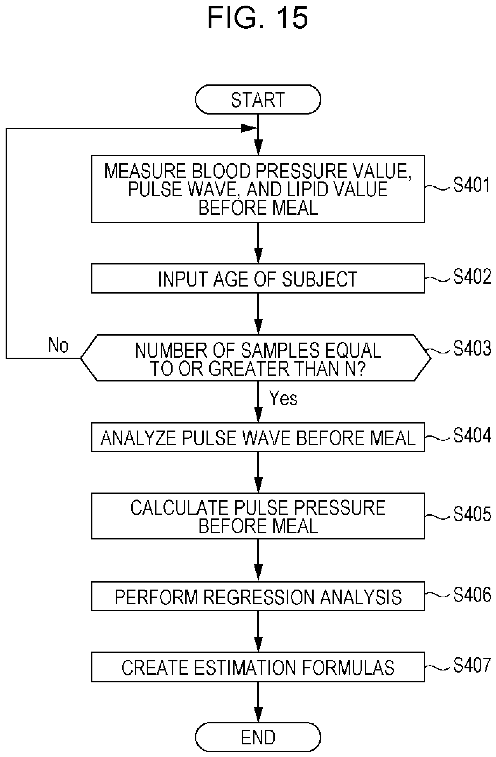

Fig. 15 is a flowchart for creating the estimation formulas used by an electronic device 1 according to another embodiment. Also in this embodiment, the estimation formulas are created by performing regression analysis on the basis of sample data. In this embodiment, the estimation formulas are created based on, as sample data, the pulse wave, the lipid value, and the blood pressure value before a meal. In this embodiment, the term before a meal refers to a time period during which the subject is fasting. The term after a meal refers to a time period during which the lipid value rises after a predetermined time period elapses after a meal (for example, about three hours after the start of the meal). In the creation of estimation formulas, in particular, estimation formulas are created by performing regression analysis using sample data for which variations in lipid value are close to a normal distribution, thereby making it possible to estimate the lipid value of the subject being examined at any timing, regardless of before or after a meal.

In the creation of the estimation formulas, first, information related to the blood pressure value, the pulse wave, and the lipid value of the subject before a meal, which are measured with a sphygmomanometer, a pulse wave meter, and a lipid measurement device, respectively, is input to the estimation formula creation device (step S401).

The age of the subject in each piece of sample data is also input to the estimation formula creation device (step S402).

The estimation formula creation device determines whether the number of samples in the sample data input in step S401 and step S402 is equal to or greater than N, which is sufficient to perform regression analysis (step S403). The number of samples N can be determined as necessary and can be set to, for example, 100. If it is determined that the number of samples is less than N (in the case of No), the estimation formula creation device repeatedly performs step S401 and step S402 until the number of samples becomes equal to or greater than N. On the other hand, if it is determined that the number of samples becomes equal to or greater than N (in the case of Yes), the estimation formula creation device proceeds to step S404 and calculates the estimation formulas.

In the calculation of the estimation formulas, the estimation formula creation device analyzes the input pulse wave before the meal (step S404). In this embodiment, the estimation formula creation device analyzes the rising index SI, the AI, and the pulse rate PR for the pulse wave before the meal. The estimation formula creation device may perform FFT analysis as the analysis of the pulse wave.

Further, the estimation formula creation device calculates the pulse pressure before the meal on the basis of the input blood pressure value before the meal (step S405).

Then, the estimation formula creation device performs regression analysis (step S406). The objective variable in the regression analysis is the lipid value before a meal. The explanatory variable in the regression analysis is the age input in step S502, the rising index SI, the AI, and the pulse rate PR for the pulse wave before the meal analyzed in step S504, and the pulse pressure before the meal calculated in step S405. When the estimation formula creation device performs FFT analysis in step S404, the explanatory variable may be, for example, the Fourier coefficients calculated as a result of the FFT analysis.

The estimation formula creation device creates estimation formulas for estimating a lipid value before a meal on the basis of the result of the regression analysis (step S407).

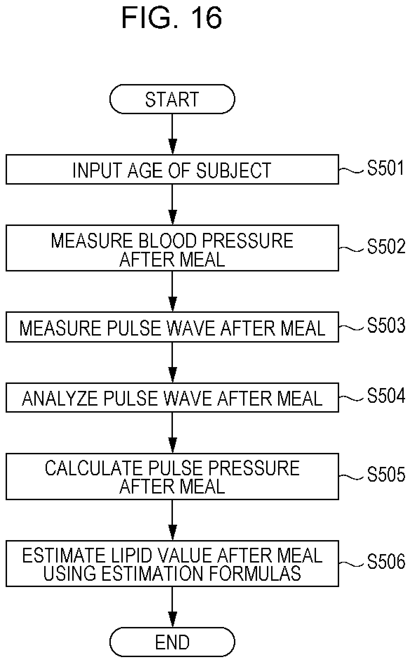

Next, a flowchart for estimating the lipid value of the subject using the estimation formulas will be described. Fig. 16 is a flowchart for estimating the lipid value of the subject using the estimation formulas created through the flowchart illustrated in Fig. 15.

First, the age of the subject is input to the electronic device 1 in response to an operation of the input unit 20 by the subject (step S501).

Then, after the subject has eaten a meal, the electronic device 1 measures the blood pressure value of the subject after the meal in response to an operation by the subject (step S502).

Further, the electronic device 1 measures the pulse wave of the subject after the meal in response to an operation by the subject (step S503).

Then, the electronic device 1 analyzes the measured pulse wave (step S504). Specifically, the electronic device 1 analyzes, for example, the rising index SI, the AI, and the pulse rate PR related to the measured pulse wave.

Further, the electronic device 1 calculates the pulse pressure after the meal on the basis of the measured blood pressure value after the meal (step S505).

The electronic device 1 applies the rising index SI, the AI, and the pulse rate PR analyzed in step S504, the pulse pressure after the meal calculated in step S505, and the age of the subject to the estimation formulas created through the flowchart in Fig. 15 to estimate the lipid value of the subject after the meal (step S506). The subject is notified of the estimated lipid value after the meal by, for example, the notification unit 60 of the electronic device 1.

In this way, the electronic device 1 estimates the lipid value after a meal on the basis of the measured blood pressure value after the meal. The electronic device 1 according to this embodiment estimates the lipid value using the blood pressure value after the meal. In particular, the AI is a parameter that can depend on the blood pressure value, and, as with the electronic device 1, the lipid value is estimated based on estimation formulas created such that the blood pressure value is included as an explanatory variable. Thus, the estimation accuracy of the lipid value can be improved.

The electronic device 1 may estimate the lipid value of the subject at any timing, instead of estimating the lipid value after a meal. The electronic device 1 can also estimate the lipid value at any timing in a non-invasive manner and in a short time.

As in the embodiment described above, the electronic device 1 according to this embodiment may update the estimation formulas stored in the storage unit 40 on the basis of the blood pressure value and the pulse wave of the subject after the meal acquired in step S502 during the estimation of the lipid value. Accordingly, the estimation formulas are updated each time the subject estimates a lipid value, and the estimation accuracy of the lipid value after a meal using the estimation formulas is improved.

(Second Embodiment)

In the first embodiment described above, the internal pressure of the cuff 72 is increased in (1) the first pressurization operation, and then the state of glucose metabolism or lipid metabolism of the subject is estimated based on the internal pressure of the cuff 72 detected by the pressure sensor 78 during (2) the depressurization operation. Recent sphygmomanometers include a sphygmomanometer of a type for measuring the blood pressure of the subject while the internal pressure of the cuff is increased. In a second embodiment, the pulse wave of the subject can be detected using a sphygmomanometer configured to measure the blood pressure of the subject while the internal pressure of the cuff is increased. That is, in the second embodiment, the state of glucose metabolism or lipid metabolism of the subject is estimated based on the internal pressure of the compression portion detected with a pressure sensor while the pressure regulation unit increases the internal pressure of the compression portion.

The second embodiment is obtained by modifying a portion of the processing in the first embodiment described above. An electronic device according to the second embodiment can have a configuration similar to that of the electronic device 1 according to the first embodiment described above. In the following, description overlapping that of the first embodiment described above will be simplified or omitted, as necessary.

In the second embodiment, the electronic device 1 estimates the state of glucose metabolism or lipid metabolism of the subject on the basis of the internal pressure of the cuff 72 detected by the pressure sensor 78 while the internal pressure of the cuff 72 is increased in (1) the first pressurization operation. In this case, the electronic device 1 may estimate (measure) the blood pressure value using the oscillometric method.

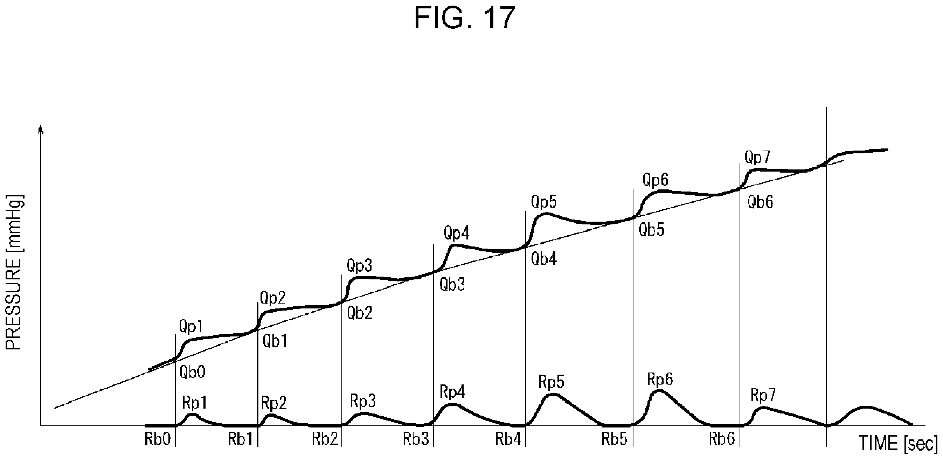

Fig. 17 is a graph illustrating an example of a change in the internal pressure of the cuff 72, which is detected by the pressure sensor 78 during (1) the first pressurization operation described above, with respect to time. In Fig. 17, as in Fig. 2 and Fig. 3, the horizontal axis represents elapsed time [seconds], and the vertical axis represents the pressure (the internal pressure of the cuff 72) [mmHg] detected by the pressure sensor 78.

As indicated by the curve in the upper portion of Fig. 17, the internal pressure of the cuff 72 detected by the pressure sensor 78 repeatedly increases and decreases slightly over time due to pulsation of the subject. As indicated by the curve in the upper portion of Fig. 17, furthermore, the internal pressure of the cuff 72 detected by the pressure sensor 78 tends to gradually increase as a whole because of the pressurization by the pressurizing pump 74.

When an increase in blood flow due to pulsation of the subject causes an expansion of the blood vessels, the internal pressure of the cuff 72 detected by the pressure sensor 78 increases. The pressure sensor 78 detects an expansion of the blood vessels caused by pulsation of the subject, such as peaks Qp1, Qp2, ..., and Qp7 of the curve in the upper portion of Fig. 17, as an increase in the internal pressure of the cuff 72 detected by the pressure sensor 78. In contrast, when a decrease in blood flow due to pulsation of the subject causes a contraction of the blood vessels, the internal pressure of the cuff 72 detected by the pressure sensor 78 slightly decreases. The pressure sensor 78 detects a contraction of the blood vessels caused by pulsation of the subject, such as bottoms Qb1, Qb2, ..., and Qb6 of the curve in the upper portion of Fig. 17, as a decrease in the internal pressure of the cuff 72 detected by the pressure sensor 78.

The curve depicted in the upper portion of Fig. 17 indicates a combination of the change in the internal pressure of the cuff 72 caused by the pulsation of the subject and the increase in the internal pressure of the cuff 72 caused by the pressurization by the pressurizing pump 74. In the second embodiment, accordingly, the control unit 10 may correct the curve indicating the change in the internal pressure of the cuff 72 with respect to time, as indicated by the curve in the upper portion of Fig. 17, in accordance with the influence of the internal pressure of the cuff 72 caused by the pressurization by the pressurizing pump 74. For example, the control unit 10 may perform correction so that a pressure equal to the amount by which the internal pressure of the cuff 72 is increased due to the pressurization by the pressurizing pump 74 is subtracted from the internal pressure of the cuff 72.

The curve depicted in the lower portion of Fig. 17 is a graph illustrating an example correction of the change in the internal pressure of the cuff 72 with respect to time in the curve depicted in the upper portion of Fig. 17. That is, the curve depicted in the upper portion of Fig. 17 indicates a change in the internal pressure of the cuff 72 with respect to time before the correction. In contrast, the curve depicted in the lower portion of Fig. 17 indicates a corrected change in the internal pressure of the cuff 72 with respect to time.

As illustrated in Fig. 17, the control unit 10 may perform correction so that, for example, the pressure corresponding to the amount by which the internal pressure of the cuff 72 is increased due to the pressurization by the pressurizing pump 74 is subtracted from the internal pressure of the cuff 72. As described above, the internal pressure of the cuff 72 detected by the pressure sensor 78 tends to gradually increase as a whole due to the influence of the pressurization by the pressurizing pump 74. Considering only the action of the pressurization by the pressurizing pump 74, it is expected that the internal pressure of the cuff 72 increases substantially linearly or in a gentle curve over time. Accordingly, the control unit 10 may correct the internal pressure of the cuff 72, which gradually increases due to the pressurization by the pressurizing pump 74, such that the internal pressure can be decreased by the amount corresponding to the increase.

As indicated by the curve in the upper portion of Fig. 17, the bottoms Qb0, Qb1, Qb2, ..., and Qb6 of the curve before the correction gradually increase. Thus, in this case, the control unit 10 may approximate the change (increase) in the values of the point Qb0, the point Qb1, the point Qb2, ..., and Qb6 with, for example, a straight line, line segments, a curve, or the like. For example, the control unit 10 may linearly approximate all of the point Qb0, the point Qb1, the point Qb2, etc. with a single straight line. Alternatively, the control unit 10 may approximate the interval between the point Qb0 and the point Qb1, the interval between the point Qb1 and the point Qb2, the interval between the point Qb2 and the point Qb3, the interval between the point Qb3 and the point Qb4, and the like with line segments. For example, the control unit 10 may perform curve approximation (curve fitting) on the point Qb0, the point Qb1, the point Qb2, etc. The control unit 10 may perform correction such that the change in pressure with respect to time (increase over time), which is obtained by the approximation described above, is subtracted from the value of the internal pressure of the cuff 72 detected by the pressure sensor 78. As a result, the values of the bottoms Qb0, Qb1, Qb2, etc. become the same (or substantially the same) value after the correction.

In the curve in the upper portion of Fig. 17, the bottoms Qb0, Qb1, Qb2, etc. of the curve before the correction are represented as, in the curve in the lower portion of Fig. 17, bottoms Rb0, Rb1, Rb2, etc. of a curve after the correction, respectively. The bottoms Rb0, Rb1, Rb2, etc. of the curve after the correction depicted in the lower portion of Fig. 17 have the same (or substantially the same) value. Fig. 17 illustrates correction such that the value of the bottom Qb0 of the curve before the correction depicted in the upper portion becomes the bottom Rb0 (zero) of the curve after the correction depicted in the lower portion. That is, Fig. 17 illustrates that the curve before the correction (upper portion) is corrected in accordance with the pressurization by the pressurizing pump 74 and that further correction is performed to make the bottoms of the curve equal to (substantially) zero to obtain the curve after the correction (lower portion).

In this way, in the second embodiment, the control unit 10 may correct the change in the internal pressure of the cuff 72 detected by the pressure sensor 78 in accordance with the pressurization by the pressure regulation unit (74, 76). Through the correction described above, the electronic device 1 according to the second embodiment can obtain a pulse wave caused by pulsation of the subject, such as the curve after the correction depicted in the lower portion of Fig. 17. The electronic device 1 according to the second embodiment may estimate the state of glucose metabolism or lipid metabolism of the subject on the basis of the pulse wave of the subject obtained in the way described above.

In this way, also in the second embodiment, the control unit 10 may estimate the state of glucose metabolism or lipid metabolism of the subject on the basis of the pulse wave having the largest peak among the pulse waves of the subject. With respect to the other points, the second embodiment can be implemented in a manner similar to that of the first embodiment.

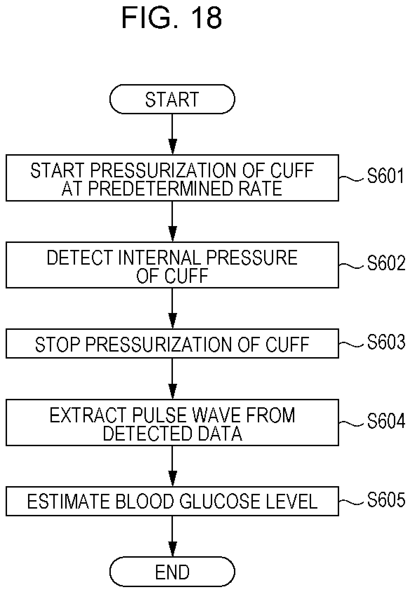

Next, the operation of the electronic device 1 according to the second embodiment will be described. Fig. 18 is a flowchart illustrating an operation of estimating the state of glucose metabolism or lipid metabolism of the subject using the electronic device 1 according to the second embodiment.

When the operation illustrated in Fig. 18 is started, the control unit 10 controls the pressurizing pump 74 to start increasing the internal pressure of the cuff 72 at a predetermined rate (step S601).

When the internal pressure of the cuff 72 is started to be increased in step S601, the control unit 10 detects the internal pressure of the cuff 72 using the pressure sensor 78 (step S602) .

When the detection of the internal pressure of the cuff 72 in step S602 is completed, the control unit 10 controls the pressurizing pump 74 to stop increasing the internal pressure of the cuff 72 (step S603). The trigger upon which the process proceeds from step S602 to step S603 may be, for example, the point in time at which a predetermined time period has elapsed. The trigger upon which the process proceeds from step S602 to step S603 may be, for example, the point in time at which the internal pressure of the cuff 72 detected by the pressure sensor 78 has reached a predetermined pressure. The trigger upon which the process proceeds from step S602 to step S603 may be, for example, the point in time at which at least one pulse wave has been detected a predetermined number of times. At the point in time at which the operation up to step S603 is completed, the control unit 10 can obtain the change in the internal pressure of the cuff 72 with respect to time as illustrated in, for example, Fig. 17.

When the increase in the internal pressure of the cuff 72 is stopped in step S603, the control unit 10 extracts a pulse wave on the basis of a change in the internal pressure of the cuff 72 detected in step S602 with respect to time (step S604). In step S604, the control unit 10 extracts a pulse wave, as indicated by, for example, the curve in the lower portion of Fig. 17, from, for example, the change in the internal pressure of the cuff 72 with respect to time as indicated by the curve in the upper portion of Fig. 17.

When the pulse wave is extracted in step S604, the control unit 10 estimates, for example, the glucose metabolism of the subject, such as the blood glucose level, on the basis of the extracted pulse wave (step S605). In step S605, the control unit 10 may estimate, for example, the lipid metabolism of the subject, such as the lipid value, instead of the glucose metabolism of the subject or together with the glucose metabolism of the subject. The method for estimating the blood glucose level or the like on the basis of a pulse wave, as performed in step S605, may be performed in a way similar to that of the first embodiment described above.

In this way, in the second embodiment, the control unit 10 causes the pressure sensor 78 to detect the internal pressure of the cuff 72 while the pressure regulation unit (74, 76) increases the internal pressure of the cuff 72. Further, the control unit 10 may estimate the state of glucose metabolism or lipid metabolism of the subject on the basis of the internal pressure of the cuff 72 detected by the pressure sensor 78. The control unit 10 may estimate a blood glucose level as the glucose metabolism of the subject, or may estimate a lipid value as the lipid metabolism of the subject.

According to the second embodiment, therefore, since only (1) the first pressurization operation described above is performed, the time required to estimate the state of glucose metabolism or lipid metabolism of the subject is relatively short. Also in the second embodiment, since the internal pressure of the cuff 72 is detected only once in the pressurized state, the physical and psychological burden on the subject, the efforts of detection, and the like are also relatively small. That is, in the electronic device 1 according to the second embodiment, the time required to estimate the state of glucose metabolism or lipid metabolism of the subject is shortened, and the burden imposed on the subject is also reduced. The electronic device 1 according to the second embodiment can thus increase usability.

As described above, the electronic device 1 can acquire the blood pressure value of the subject using a conventionally known method, for example. Accordingly, in addition to performing the operation described above, the control unit 10 may further determine the blood pressure value of the subject on the basis of the internal pressure of the cuff 72 detected by the pressure sensor 78, and estimate the state of glucose metabolism or lipid metabolism of the subject on the basis of the blood pressure value.

As described above in the first embodiment, the control unit 10 may estimate the state of glucose metabolism or lipid metabolism of the subject on the basis of the internal pressure of the cuff 72 detected by the pressure sensor 78 while the pressure regulation unit (74, 76) reduces the internal pressure of the cuff 72. In short, in the electronic device 1, the control unit 10 may estimate the state of glucose metabolism or lipid metabolism of the subject on the basis of the internal pressure of the cuff 72 detected by the pressure sensor 78 while the pressure regulation unit (74, 76) changes the internal pressure of the cuff 72.

(Third Embodiment)

In the first embodiment and the second embodiment described above, the state of glucose metabolism or lipid metabolism of the subject is estimated based on the internal pressure of the cuff 72 detected by the pressure sensor 78 while the pressure regulation unit (74, 76) changes the internal pressure of the cuff 72. In a third embodiment, in contrast, the pressure sensor 78 detects the internal pressure of the cuff 72 in a period during which the pressure regulation unit (74, 76) maintains the internal pressure of the cuff 72 after the pressure regulation unit (74, 76) changes the internal pressure of the cuff 72. That is, in the first embodiment and the second embodiment, the internal pressure of the cuff 72 is detected while the internal pressure of the cuff 72 is changed. In the third embodiment, in contrast, the internal pressure of the cuff 72 is detected while the internal pressure of the cuff 72 is maintained.

The third embodiment is obtained by modifying a portion of the processing in the first embodiment or the second embodiment described above. An electronic device according to the third embodiment can have a configuration similar to that of the electronic device 1 according to the first embodiment or the second embodiment described above. In the following, description overlapping that of the first embodiment or the second embodiment described above will be simplified or omitted, as necessary.

In the third embodiment, before the internal pressure of the cuff 72 is maintained, the pressure regulation unit (74, 76) changes the internal pressure of the cuff 72. In this case, an electronic device 1 according to the third embodiment may change the internal pressure of the cuff 72 to any internal pressure at which the pulse wave of the subject can be detected in response to the pressure sensor 78 detecting the internal pressure of the cuff 72. For example, the electronic device 1 according to the third embodiment may change the internal pressure of the cuff 72 between the maximum blood pressure and the minimum blood pressure of the subject and then maintain the internal pressure of the cuff 72.

In this way, in the third embodiment, the pressure regulation unit (74, 76) changes the internal pressure of the cuff 72 and then maintains the internal pressure of the cuff 72. In the third embodiment, furthermore, the control unit 10 estimates the state of glucose metabolism or lipid metabolism of the subject on the basis of the internal pressure of the cuff 72 detected by the pressure sensor 78 while the pressure regulation unit (74, 76) maintains the internal pressure of the cuff 72.

According to the third embodiment, since only an operation of maintaining the internal pressure of the cuff 72 after increasing or decreasing the internal pressure of the cuff 72 is performed, the time required to estimate the state of glucose metabolism or lipid metabolism of the subject is relatively short. Also in the third embodiment, since the internal pressure of the cuff 72 is detected only once in the state in which the internal pressure is maintained, the physical and psychological burden on the subject, the efforts of detection, and the like are also relatively small. That is, in the electronic device 1 according to the third embodiment, the time required to estimate the state of glucose metabolism or lipid metabolism of the subject is shortened, and the burden imposed on the subject is also reduced. The electronic device 1 according to the third embodiment can thus increase usability.

According to the third embodiment, furthermore, the pulse wave of the subject is detected in the state where the internal pressure of the cuff 72 is maintained. Thus, effects such as stabilizing the pulse wave of the subject to be detected can be expected. For example, during the detection of the pulse wave of the subject, the internal pressure of the cuff 72 is not increased or decreased, and thus there is no need to operate the pressure regulation unit (74, 76). According to the third embodiment, therefore, there is also no need to take into account the influence of noise and the like that can occur when the pressure regulation unit (74, 76) is operated. According to the third embodiment, furthermore, the pulse wave of the subject can be detected a plurality of times under the same state. According to the third embodiment, therefore, it is possible to perform processing such as averaging the waveforms of a plurality of pulse waves, for example.

Some embodiments have been described to fully and clearly disclose the present invention. However, the appended claims are not to be limited to the embodiments described above, but are to be configured to embody all modifications and alternative configurations that may be created by a person skilled in the art in this technical field within the scope of the basic matter described herein. The requirements described in some embodiments can be combined, as desired. That is, a person skilled in the art can make various changes and modifications to the content of the present disclosure on the basis of the present disclosure. Thus, such changes and modifications are included in the scope of the present disclosure. For example, in each embodiment, each functional unit, each means, each step, and the like can be added to another embodiment or replaced with each functional unit, each means, each step, and the like of another embodiment so as not to logically contradict each other. In each embodiment, a plurality of functional units, means, steps, and the like can be combined into one or divided. In addition, the embodiments of the present disclosure described above are not limited to faithful implementations of the embodiments described above, and may be implemented by appropriately combining the features or omitting some of them.

For example, in a case where noise may occur when the pressure regulation unit (74, 76) is operated, the noise may affect measurement of the pulse wave of the subject. Specifically, in a case such as a case where the pressurizing pump 74 constituting the pressure regulation unit (74, 76) is a diaphragm pump, noise may occur when a motor that drives the pump is operated. In this case, the operating frequency of the pressure regulation unit (74, 76) and the frequency of the pulse wave of the subject may be different from each other. For example, the pulse wave of the subject is assumed to be about several Hz, and the operating frequency of the pressure regulation unit (74, 76) may be set to be about ten times as high as about several Hz. This reduces the influence of the noise that affects measurement of the pulse wave of the subject.

To describe an example estimation method based on a change in pulse wave according to the embodiments described above, an example of a pulse wave is illustrated in Fig. 8. The pulse wave illustrated in Fig. 8 is merely an example. When a pulse wave is detected in such a manner that the target region of the subject differs, a pulse wave having a different waveform from that of the pulse wave illustrated in Fig. 8 may be obtained. There are various patterns of the waveform as illustrated in Fig. 8. The waveform as illustrated in Fig. 8 may change depending on, for example, the target region of the subject (region where the pulse wave is to be measured), the form of measurement, the characteristics of the cuff, and so on. That is, the pulse wave of the subject detected by the electronic device 1 according to an embodiment may differ depending on the target region of the subject. In addition, the pulse wave of the subject detected by the electronic device 1 according to an embodiment may also differ depending on the form of measurement by the electronic device 1. In addition, the pulse wave of the subject detected by the electronic device 1 according to an embodiment may also differ depending on the configuration of the electronic device 1. Likewise, the pulse wave as illustrated in Fig. 10 is also merely an example, and various waveforms may be conceivable.

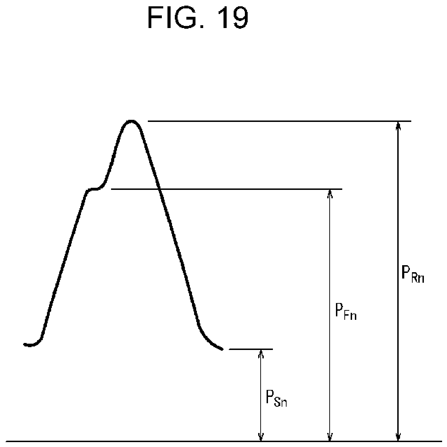

For example, in the embodiments described above, the waveform of the pulse wave as illustrated in Fig. 10 may be a waveform as illustrated in Fig. 19 depending on measurement conditions or the like. Fig. 19 is a diagram illustrating an example of a pulse wave acquired as a modification of the waveform illustrated in Fig. 10. In particular, Fig. 19 illustrates an example of a waveform predicted to be actually acquired by an electronic device according to the embodiments described above. Fig. 19 illustrates only one waveform (one wavelength) of an acquired pulse wave. In Fig. 19, the horizontal axis represents time, and the vertical axis represents the pressure. Other signs illustrated in Fig. 19 have meanings similar to those illustrated in Fig. 10. As described above, the waveform of a pulse wave acquired by an electronic device according to the embodiments described above may change in accordance with, for example, the softness of an artery of the subject, a measurement region of the subject, and/or measurement conditions or the like.

Reference Signs List

1    electronic device
10   control unit
20   input unit
30   power supply unit
40   storage unit
50   communication unit
60   notification unit
70   blood pressure measurement unit
72   cuff
74   pressurizing pump
76   exhaust valve
78   pressure sensor

**Claims**

1. An electronic device comprising:

   a compression portion that compresses a target region of a subject;
   a pressure regulation unit that regulates an internal pressure of the compression portion;
   a pressure sensor that detects the internal pressure of the compression portion; and
   a control unit that estimates a state of glucose metabolism or lipid metabolism of the subject on the basis of the internal pressure of the compression portion detected by the pressure sensor in a period during which the pressure regulation unit changes the internal pressure of the compression portion.

2. The electronic device according to Claim 1, wherein the control unit estimates a state of glucose metabolism or lipid metabolism of the subject on the basis of a pulse wave of the subject obtained by correcting a change in the internal pressure of the compression portion detected by the pressure sensor in accordance with a change made by the pressure regulation unit to the internal pressure of the compression portion.

3. The electronic device according to Claim 2, wherein the control unit estimates a state of glucose metabolism or lipid metabolism of the subject on the basis of a pulse wave of the subject obtained by correcting a change in the internal pressure of the compression portion detected by the pressure sensor without using a digital filter.

4. The electronic device according to Claim 2 or 3,
wherein the control unit estimates a state of glucose metabolism or lipid metabolism of the subject on the basis of a pulse wave having a largest peak among pulse waves of the subject.

5. The electronic device according to any of Claims 1 to 4, wherein the control unit determines a blood pressure value of the subject on the basis of the internal pressure of the compression portion detected by the pressure sensor, and estimates a state of glucose metabolism or lipid metabolism of the subject on the basis of the blood pressure value.

6. The electronic device according to any of Claims 1 to 5, wherein the control unit estimates a blood glucose level as glucose metabolism of the subject or estimates a lipid value as lipid metabolism of the subject.

7. The electronic device according to any of Claims 1 to 6, wherein the compression portion is a cuff used in a cuff-type sphygmomanometer.

8. The electronic device according to any of Claims 1 to 7, wherein the pressure regulation unit includes at least one of a pressurizing pump that increases the internal pressure of the compression portion, and an exhaust valve that reduces the internal pressure of the compression portion.

9. The electronic device according to any of Claims 1 to 8, wherein the control unit estimates a state of glucose metabolism or lipid metabolism of the subject on the basis of the internal pressure of the compression portion detected by the pressure sensor in a period during which the pressure regulation unit reduces the internal pressure of the compression portion.

10. The electronic device according to Claim 9, wherein the control unit estimates a state of glucose metabolism or lipid metabolism of the subject on the basis of the internal pressure of the compression portion detected by the pressure sensor in a period during which the pressure regulation unit reduces the internal pressure of the compression portion after the pressure regulation unit increases the internal pressure of the compression portion.

11. The electronic device according to any of Claims 1 to 8, wherein the control unit estimates a state of glucose metabolism or lipid metabolism of the subject on the basis of the internal pressure of the compression portion detected by the pressure sensor in a period during which the pressure regulation unit increases the internal pressure of the compression portion.

12. An electronic device comprising:

a compression portion that compresses a target region of a subject;
a pressure regulation unit that regulates an internal pressure of the compression portion;
a pressure sensor that detects the internal pressure of the compression portion; and
a control unit that estimates a state of glucose metabolism or lipid metabolism of the subject on the basis of the internal pressure of the compression portion detected by the pressure sensor in a period during which the pressure regulation unit maintains the internal pressure of the compression portion after the pressure regulation unit changes the internal pressure of the compression portion.

13. A method for controlling an electronic device, comprising:

a step of compressing a target region of a subject with a compression portion;
a step of regulating an internal pressure of the compression portion with a pressure regulation unit;
a step of detecting the internal pressure of the compression portion with a pressure sensor; and
a step of estimating a state of glucose metabolism or lipid metabolism of the subject on the basis of the internal pressure of the compression portion detected by the pressure sensor in a period during which the pressure regulation unit changes the internal pressure of the compression portion.

14. A program for causing a computer to execute:

a step of compressing a target region of a subject with a compression portion;
a step of regulating an internal pressure of the compression portion with a pressure regulation unit;
a step of detecting the internal pressure of the compression portion with a pressure sensor; and
a step of estimating a state of glucose metabolism or lipid metabolism of the subject on the basis of the internal pressure of the compression portion detected by the pressure sensor in a period during which the pressure regulation unit changes the internal pressure of the compression portion.

# FIG. 1

1

10

| CONTROL UNIT | ── | INPUT UNIT | ～ 20 |
| | ── | POWER SUPPLY UNIT | ～ 30 |
| | ── | STORAGE UNIT | ～ 40 |
| | ── | COMMUNICATION UNIT | ～ 50 |
| | ── | NOTIFICATION UNIT | ～ 60 |

74

78

76

| PRESSURIZING PUMP | PRESSURE SENSOR | EXHAUST VALVE |

～ 70

| CUFF |

72

## FIG. 2

EP 3 915 468 A1

# FIG. 3

EP 3 915 468 A1

FIG. 4

FIG. 5

FIG. 6

# FIG. 7

START

PRESSURIZE CUFF TO
PREDETERMINED PRESSURE — S1

START DEPRESSURIZATION OF CUFF — S2

DETECT INTERNAL PRESSURE
OF CUFF — S3

STOP DEPRESSURIZATION OF CUFF — S4

EXTRACT PULSE WAVE
FROM DETECTED DATA — S5

ESTIMATE BLOOD GLUCOSE LEVEL — S6

END

FIG. 8

FIG. 9

# FIG. 10

$$AI_n = (P_{Rn} - P_{Sn}) / (P_{Fn} - P_{Sn})$$

| PULSE | 1 | 2 | 3 | 4 | · · · · · | · · · | n |

ANGLE

$P_{Fn}$

$P_{Rn}$

$P_{Sn}$

TIME

FIG. 11A

FIG. 11B

# FIG. 12

```
            ┌─────────────┐
            │    START    │
            └─────────────┘
                   │
                   ▼
  ┌──────────────────────────────────┐
  │   INPUT BLOOD GLUCOSE LEVEL       │
  │   AND BLOOD PRESSURE VALUE        │──── S101
  │        BEFORE MEAL               │
  └──────────────────────────────────┘
                   │
                   ▼
  ┌──────────────────────────────────┐
  │   INPUT BLOOD GLUCOSE LEVEL,      │
  │     BLOOD PRESSURE VALUE,         │──── S102
  │   AND PULSE WAVE AFTER MEAL       │
  └──────────────────────────────────┘
                   │
                   ▼
   No    ╱────────────────────────────╲
  ◄──────  NUMBER OF SAMPLES EQUAL      ──── S103
         ╲   TO OR GREATER THAN N?    ╱
           ╲──────────────────────────╱
                   │ Yes
                   ▼
  ┌──────────────────────────────────┐
  │   ANALYZE PULSE WAVE AFTER MEAL   │──── S104
  └──────────────────────────────────┘
                   │
                   ▼
  ┌──────────────────────────────────┐
  │    CALCULATE PULSE PRESSURE       │
  │    DIFFERENCE BETWEEN             │──── S105
  │    BEFORE AND AFTER MEAL          │
  └──────────────────────────────────┘
                   │
                   ▼
  ┌──────────────────────────────────┐
  │   PERFORM REGRESSION ANALYSIS     │──── S106
  └──────────────────────────────────┘
                   │
                   ▼
  ┌──────────────────────────────────┐
  │    CREATE ESTIMATION FORMULAS     │──── S107
  └──────────────────────────────────┘
                   │
                   ▼
            ┌─────────────┐
            │     END     │
            └─────────────┘
```

# FIG. 13

```
                    ┌─────────────┐
                    │    START    │
                    └─────────────┘
                           │
                           ▼
        ┌────────────────────────────────────┐
        │       INPUT AGE OF SUBJECT          │～S301
        └────────────────────────────────────┘
                           │
                           ▼
        ┌────────────────────────────────────┐
        │   MEASURE BLOOD PRESSURE VALUE      │～S302
        │           BEFORE MEAL               │
        └────────────────────────────────────┘
                           │
                           ▼
        ┌────────────────────────────────────┐
        │   MEASURE BLOOD PRESSURE VALUE      │～S303
        │           AFTER MEAL                │
        └────────────────────────────────────┘
                           │
                           ▼
        ┌────────────────────────────────────┐
        │    MEASURE PULSE WAVE AFTER MEAL    │～S304
        └────────────────────────────────────┘
                           │
                           ▼
        ┌────────────────────────────────────┐
        │    ANALYZE PULSE WAVE AFTER MEAL    │～S305
        └────────────────────────────────────┘
                           │
                           ▼
        ┌────────────────────────────────────┐
        │      CALCULATE PULSE PRESSURE       │
        │        DIFFERENCE BETWEEN           │～S306
        │       BEFORE AND AFTER MEAL         │
        └────────────────────────────────────┘
                           │
                           ▼
        ┌────────────────────────────────────┐
        │  ESTIMATE BLOOD GLUCOSE LEVELS      │
        │      BEFORE AND AFTER MEAL          │～S307
        │   USING ESTIMATION FORMULAS         │
        └────────────────────────────────────┘
                           │
                           ▼
                    ┌─────────────┐
                    │     END     │
                    └─────────────┘
```

# FIG. 14

# FIG. 15

```
        ┌─────────────┐
        │    START    │
        └─────────────┘
               │
  ┌────────────┼──────────────────────────┐
  │            ▼                           │
  │  ┌─────────────────────────┐          │
  │  │ MEASURE BLOOD PRESSURE  │          │
  │  │ VALUE, PULSE WAVE, AND  │───S401   │
  │  │ LIPID VALUE BEFORE MEAL │          │
  │  └─────────────────────────┘          │
  │            │                           │
  │            ▼                           │
  │  ┌─────────────────────────┐          │
  │  │  INPUT AGE OF SUBJECT   │───S402   │
  │  └─────────────────────────┘          │
  │            │                           │
  │            ▼                           │
  │  No ╱───────────────────────╲         │
  └────┤ NUMBER OF SAMPLES EQUAL │───S403
       ╲ TO OR GREATER THAN N?  ╱
        ╲───────────────────────╱
               │ Yes
               ▼
  ┌────────────────────────────────┐
  │ ANALYZE PULSE WAVE BEFORE MEAL │───S404
  └────────────────────────────────┘
               │
               ▼
  ┌────────────────────────────────┐
  │    CALCULATE PULSE PRESSURE    │───S405
  │          BEFORE MEAL           │
  └────────────────────────────────┘
               │
               ▼
  ┌────────────────────────────────┐
  │  PERFORM REGRESSION ANALYSIS   │───S406
  └────────────────────────────────┘
               │
               ▼
  ┌────────────────────────────────┐
  │   CREATE ESTIMATION FORMULAS   │───S407
  └────────────────────────────────┘
               │
               ▼
        ┌─────────────┐
        │     END     │
        └─────────────┘
```

# FIG. 16

```
              ┌─────────────┐
              │    START    │
              └──────┬──────┘
                     │
                     ▼
      ┌──────────────────────────────┐
      │     INPUT AGE OF SUBJECT      │── S501
      └──────────────┬───────────────┘
                     │
                     ▼
      ┌──────────────────────────────┐
      │    MEASURE BLOOD PRESSURE     │── S502
      │          AFTER MEAL           │
      └──────────────┬───────────────┘
                     │
                     ▼
      ┌──────────────────────────────┐
      │  MEASURE PULSE WAVE AFTER MEAL │── S503
      └──────────────┬───────────────┘
                     │
                     ▼
      ┌──────────────────────────────┐
      │  ANALYZE PULSE WAVE AFTER MEAL │── S504
      └──────────────┬───────────────┘
                     │
                     ▼
      ┌──────────────────────────────┐
      │     CALCULATE PULSE PRESSURE  │── S505
      │          AFTER MEAL           │
      └──────────────┬───────────────┘
                     │
                     ▼
      ┌──────────────────────────────┐
      │ ESTIMATE LIPID VALUE AFTER MEAL│── S506
      │    USING ESTIMATION FORMULAS   │
      └──────────────┬───────────────┘
                     │
                     ▼
              ┌─────────────┐
              │     END     │
              └─────────────┘
```

# FIG. 17

EP 3 915 468 A1

# FIG. 18

```
        ┌─────────────┐
        │    START    │
        └──────┬──────┘
               │
               ▼
┌───────────────────────────────┐
│ START PRESSURIZATION OF CUFF  │─── S601
│   AT PREDETERMINED RATE       │
└───────────────┬───────────────┘
               │
               ▼
┌───────────────────────────────┐
│    DETECT INTERNAL PRESSURE   │─── S602
│          OF CUFF              │
└───────────────┬───────────────┘
               │
               ▼
┌───────────────────────────────┐
│  STOP PRESSURIZATION OF CUFF  │─── S603
└───────────────┬───────────────┘
               │
               ▼
┌───────────────────────────────┐
│    EXTRACT PULSE WAVE FROM     │─── S604
│         DETECTED DATA          │
└───────────────┬───────────────┘
               │
               ▼
┌───────────────────────────────┐
│  ESTIMATE BLOOD GLUCOSE LEVEL  │─── S605
└───────────────┬───────────────┘
               │
               ▼
        ┌─────────────┐
        │     END     │
        └─────────────┘
```

# FIG. 19

<table>
<tr><td colspan="2"><strong>INTERNATIONAL SEARCH REPORT</strong></td><td>International application No.<br>PCT/JP2020/000059</td></tr>
</table>

| A. CLASSIFICATION OF SUBJECT MATTER |
|---|
| A61B 5/02(2006.01)i; A61B 5/022(2006.01)i; A61B 5/145(2006.01)i<br>FI: A61B5/02 310J; A61B5/022 400F; A61B5/022 Z; A61B5/145; A61B5/022 400E |

According to International Patent Classification (IPC) or to both national classification and IPC

| B. FIELDS SEARCHED |
|---|
| Minimum documentation searched (classification system followed by classification symbols)<br>A61B5/02; A61B5/022; A61B5/145 |

Documentation searched other than minimum documentation to the extent that such documents are included in the fields searched
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| Y | JP 2018-183461 A (KYOCERA CORP.) 22.11.2018 (2018-11-22) paragraphs [0045], [0097]-[0107], fig. 11-13 | 1-14 |
| Y | JP 2013-90825 A (OMRON HEALTHCARE CO., LTD.) 16.05.2013 (2013-05-16) paragraphs [0002]-[0004], [0015]-[0040], fig. 1-4 | 1-14 |
| Y | JP 2004-180910 A (OMRON HEALTHCARE CO., LTD.) 02.07.2004 (2004-07-02) paragraphs [0075]-[0077], fig. 9 | 3 |
| Y | JP 2010-142418 A (OMRON HEALTHCARE CO., LTD.) 01.07.2010 (2010-07-01) paragraphs [0072]-[0081], fig. 9 | 12 |

☒ Further documents are listed in the continuation of Box C.  ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>19 February 2020 (19.02.2020) | Date of mailing of the international search report<br>03 March 2020 (03.03.2020) |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

| International application No. |
|---|
| PCT/JP2020/000059 |

C (Continuation). DOCUMENTS CONSIDERED TO BE RELEVANT

| Category* | Citation of document, with indication, where appropriate, of the relevant passages | Relevant to claim No. |
|---|---|---|
| A | CN 105686815 A (YANG, Hang) 22.06.2016 (2016-06-22) entire text, all drawings | 1-14 |
| A | CN 203861234 U (JILIN UNIVERSITY) 08.10.2014 (2014-10-08) entire text, all drawings | 1-14 |

Form PCT/ISA/210 (continuation of second sheet) (January 2015)

INTERNATIONAL SEARCH REPORT

Information on patent family members

International application No.

PCT/JP2020/000059

| Patent Documents referred in the Report | Publication Date | Patent Family | Publication Date |
|---|---|---|---|
| JP 2018-183461 A | 22 Nov. 2018 | (Family: none) | |
| JP 2013-90825 A | 16 May 2013 | US 2014/0257116 A1 paragraphs [0002]-[0004], [0050]-[0078], fig. 1-4 CN 103889320 A | |
| JP 2004-180910 A | 02 Jul. 2004 | US 2004/0147848 A1 paragraphs [0093]-[0096], fig. 9 EP 1426008 A1 CN 1504165 A | |
| JP 2010-142418 A | 01 Jul. 2010 | US 2011/0251499 A1 paragraphs [0096]-[0107], fig. 9 CN 102256539 A | |
| CN 105686815 A | 22 Jun. 2016 | (Family: none) | |
| CN 203861234 U | 08 Oct. 2014 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019008542 A
- JP 2019091652 A
- JP 2002360530 A